(19) **Europäisches Patentamt / European Patent Office / Office européen des brevets**

(11) **EP 4 785 951 A1**

(12) **EUROPEAN PATENT APPLICATION**
published in accordance with Art. 153(4) EPC

(43) Date of publication:
05.08.2026 Bulletin 2026/32

(21) Application number: 24870409.0

(22) Date of filing: 06.09.2024

(51) International Patent Classification (IPC):
$A61K\ 39/12^{(2006.01)}$ $C07K\ 14/01^{(2006.01)}$
$C12N\ 15/34^{(2006.01)}$ $A61P\ 31/20^{(2006.01)}$

(52) Cooperative Patent Classification (CPC):
A61K 39/12; A61P 31/20; C07K 14/01

(86) International application number:
PCT/CN2024/117421

(87) International publication number:
WO 2025/066863 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 CN 202311270370
27.09.2023 PCT/CN2023/122229

(71) Applicant: SHANGFUSHAFEI (SHANGHAI)
BIOTECHNOLOGY CO., LTD
New Area Shanghai 201318 (CN)

(72) Inventors:
• XU, Jianqing
Shanghai 200032 (CN)
• ZHANG, Xiaoyan
Shanghai 200032 (CN)
• DONG, Lanlan
Shanghai 200032 (CN)

(74) Representative: Pfenning, Meinig & Partner mbB
Patent- und Rechtsanwälte
Theresienhöhe 11a
80339 München (DE)

Remarks:
The complete document including Reference
Table(s) and the Sequence Listing(s) can be
downloaded from the EPO website

(54) **AFRICAN SWINE FEVER VIRUS IMMUNOGEN COMBINATION AND USE THEREOF**

(57) Provided are an African swine fever virus immunogen combination and a use thereof. An immunogenic composition, comprising: (A) an antibody immunogen group containing immunogens derived from African swine fever virus structural proteins; and (B) a recombinant T cell immunogen group containing immunogens derived from non-structural proteins of African swine fever virus. Also provided are an encoding nucleic acid molecule of the immunogenic composition, a vector and a host cell thereof, a vaccine containing one or more of the foregoing, and a related product. The two immunogen combinations can effectively induce binding antibodies against p72, p54 and p30, as well as activate T cell responses against T antigens, p 17 and penton, thereby playing an effective preventive role against African swine fever virus.

**Description**

**TECHNICAL FIELD**

This invention relates to the fields of vaccines and biotechnology. Specifically, this application relates to a dual immunogen composition comprising an antibody immunogen composed of structural proteins of African swine fever virus and a T-cell immunogen derived from non-structural proteins, a vaccine comprising the immunogen composition, and use thereof.

**BACKGROUND**

African swine fever is an infectious disease caused by African swine fever virus (ASFV). Its main hosts are domestic pigs and wild boars such as warthogs, bush boars, and giant forest hogs, and the ASFV can be transmitted via soft ticks of the genus *Ornithodoros*.

The ASFV is a large, double-stranded DNA enveloped virus, with viral particles reaching up to 200 nm in diameter. As the sole member of the family *Asfarviridae* (African swine fever virus), it has a genome ranging from 170 kb to 194 kb in size, encoding about 160 proteins including structural proteins and host immune regulatory proteins. Based on the gene sequence encoding the major capsomere protein of the virus (B646L), ASFV can be categorized into 24 genotypes. Due to host differences and strain virulence, ASF infection manifests diverse clinical symptoms. Domestic pigs infected with highly virulent strains typically exhibit symptoms such as high fever, cutaneous cyanosis, and multiple organ hemorrhage, and die within 3-7 days after infection, with a mortality rate approaching 100%.

African swine fever was initially confined to African countries. Since the 1960s, it has successively spread to and affected regions such as Europe, South America and Asia. After being introduced to China in August 2018, it caused huge economic losses to China and severely impacted the domestic breeding industry. According to data from the World Organisation for Animal Health (WOAH), African swine fever has now spread to all five continents, and more and more countries are reporting outbreaks. [1]

Developing safe and effective African swine fever vaccines is an effective means of preventing the outbreak and spread of African swine fever. Due to the complexity of the viral structure and the lack of research on the host's immune mechanism against infection, research on African swine fever vaccines faces great challenges. Antibody responses against African swine fever virus can be detected in recovered pigs, but passively immunized healthy pigs cannot resist viral invasion. Oura et al. also found that depletion of CD8+ T cells in pigs immunized with an attenuated vaccine using antibodies would abolish the inherent protective efficacy.[2] Inactivated African swine fever virus vaccines cannot stimulate sufficient immune responses and have been proven unable to confer effective protection. Live attenuated vaccines can replicate *in vivo* after immunization, simulating viral infection under natural conditions. Immunization with live attenuated vaccines obtained through tissue passage culture or natural screening, such as NH/P68 and OURT88/3, can induce immune protection against the same genotype strain in experimental pigs, but lack cross-protection against heterologous genotype strains.

In recent years, more and more genes associated with the virulence of ASFV and evasion of host immune responses have been identified, and live attenuated vaccines developed by deleting such genes have emerged continuously. After knockout of the 9GL and UK genes of the Georgia 2007 strain, experimental pigs were able to resist challenge with Georgia 2007 on Day 14 post-immunization, whereas knockout of the same genes in the Chinese epidemic strain HLJ/2008 failed to provide protection. [3]In addition, there are still several critical problems with live attenuated vaccines:

- Problem (1): Safety. The mechanism by which African swine fever virus resists clearance by host cells is highly complex and involves multiple signaling pathways. Deleting a limited number of genes cannot guarantee its safety. Many live attenuated vaccines will show virulence reversion during continuous passage and are often accompanied by more serious side effects. The live attenuated vaccine HLJ/-18-6GD developed by the Harbin Veterinary Research Institute team[4], with a total of six genes deleted from the MGF306/505 family, can provide 100% protection against highly pathogenic homologous strains, but reversion to virulence was observed in safety tests, and only after further knockout of the CD2v gene was its virulence further attenuated. In July 2023, Vietnam approved two live attenuated vaccines for domestic marketing. However, there is a lack of sufficient safety data. The WOAH issued a warning that "there is a risk in using substandard vaccines" [1]

- Problem (2): Broad-spectrum protection. Currently, protective live attenuated vaccines are only effective against homologous strains. Given the large number of ASFV genotypes and serotypes, broad-spectrum immune responses can only be achieved by identifying key conserved T-cell epitopes.

- Problem (3): Restricted large-scale production. The target cells of ASFV are primary porcine macrophages, which are difficult to use for commercial large-scale production.

With the deepening of research on antigens for viral immune protection, research on screening suitable combinations of immunogens and developing vaccines using different vaccine platforms such as subunit vaccines, nucleic acid vaccines, and viral vector vaccines is widely carried out. Gomez-Puertas et al. found that p72, p54, and p30 proteins can induce the body to produce neutralizing antibodies, and there are countless subunit vaccines and DNA vaccines focusing on combinations of these three immunogens.[5] Immunization of experimental pigs with p54 protein, p30 protein, or a p54 and p30 fusion protein expressed separately using a baculovirus expression system can induce neutralizing antibodies and protect against strain E75. However, the combination of p72, p54, and p30 fails to protect experimental pigs against challenge with Pr4 strain. CD2v, as the only protein on the outer membrane of ASFV, can mediate the red blood cell agglutination of ASFV *in vitro* and is an important indicator for identifying ASFV serotypes. Immunization with CD2v protein alone can mediate partial protection against the E75 strain, but this result cannot be replicated on other strains. Fusion expression of the extracellular domain of CD2v with p54 and p30, respectively, using a DNA vaccine platform can induce strong binding antibodies and T-cell responses, yet provides no protection upon challenge. Further immunization after fusion with ubiquitin protein induces strong T-cell responses but fails to induce the protection of binding and neutralizing antibodies, resulting in only partial protection upon challenge, wherein only 2 out of 6 experimental pigs survived (2/6 survival).

Lacasta et al. constructed a random DNA vaccine library containing 80 coding frames of ASFV fused with ubiquitin protein, but it only produced partial protection. [6] Adenovirus and vaccinia virus are major viral vectors for African swine fever vaccine development; however, most studies only detect viral antigen-related immune responses without evaluating protective efficacy against challenge. Expression of A151R, B119L, B602L, EP402R∆PRR, B438L, K205R, A104R, pp62, and p72 by adenovirus, combined with BioMize adjuvant, can induce higher levels of binding antibodies, but challenge results showed not only no protection but even exacerbated clinical symptoms in the experimental group. Expression of p32, p54, pp62, p72, p37-34-14, p150-I, and p150-II by adenovirus combined with BioMize adjuvant also induces strong humoral immune responses, yet only 2 out of 10 experimental pigs survived challenge (2/10). Immunization with the same immunogen combination combined with ZTS-01 adjuvant achieved better protective efficacy (5/9 survival), despite inducing lower antibody levels.

In addition to single viral vectors, numerous studies have explored the feasibility of prime-boost immunization regimens using different viral vectors, or viral vectors in combination with subunit vaccines and DNA vaccines. Priming with adenovirus expressing p72, p54, p30, B602L, E199L, EP153R, F317L, and MGF505-5R, followed by boosting with vaccinia virus expressing the same immunogens, resulted in 100% survival of experimental animals after challenge with OUR T88/1. However, viral loads in surviving animals remained high 20 days post-challenge, and infectivity could not be ruled out.[7]

In summary, there remains an urgent need in the art to further explore the infection mechanism of African swine fever virus and host immune protective mechanisms, identify and screen immunogens or antigenic epitopes capable of inducing immune responses, and develop safe and effective African swine fever vaccines.

As a widely used vaccine vector, vaccinia virus can induce high levels of humoral and cellular immune responses. Vaccinia virus was originally developed against smallpox virus, and large-scale human vaccination has confirmed its high safety profile. Furthermore, vaccinia virus can accommodate exogenous genes up to 30 kb in length.

ASFV has a complex structure and can resist various extreme physical environments. It is transmitted via contaminated equipment, swill, feed, or attachment to pork products. At present, the main measures to control African swine fever in China remain control, management, and culling. Given China's huge pig breeding industry, dominated by small-scale backyard farming with weak biosecurity awareness, African swine fever will persist for a long time and may re-emerge at any time. Therefore, the development of safe and effective African swine fever vaccines is urgently imperative.

References:

[1] Health W O F A. AFRICAN SWINE FEVER (ASF) Situation Report 55[R]. 2024.

[2] Oura C A, Denyer M, Takamatsu H, et al. In vivo depletion of CD8+ T lymphocytes abrogates protective immunity to African swine fever virus[J]. Journal of General Virology, 2005, 86(9): 2445-2450

[3] Wu K, Liu J, Wang L, et al. Current state of global African swine fever vaccine development under the prevalence and transmission of ASF in China[J]. Vaccines, 2020, 8(3): 531.DOI:10.3390/vaccines8030531.

[4] Chen W, Zhao D, He X, et al. A seven-gene-deleted African swine fever virus is safe and effective as a live attenuated vaccine in pigs[J]. Sci China Life Sci, 2020, 63(5): 623-634.DOI:10.1007/s11427-020-1657-9.

[5] Gomez-Puertas P, Rodriguez F, Oviedo J M, et al. Neutralizing antibodies to different proteins of African swine fever virus inhibit both virus attachment and internalization[J]. Journal of virology, 1996, 70(8): 5689-5694

[6] Lacasta A, Ballester M, Monteagudo P L, et al. Expression library immunization can confer protection against lethal challenge with African swine fever virus[J]. Journal of virology, 2014, 88(22): 13322-13332

[7] Goatley L C, Reis A L, Portugal R, et al. A pool of eight virally vectored African swine fever antigens protect pigs against fatal disease[J]. Vaccines, 2020, 8(2): 234

## SUMMARY OF THE INVENTION

The present application addresses a pressing technical problem in the field by developing a novel African swine fever vaccine.

In some aspects of the present application, an immunogenic composition is provided comprising:

(A) An antibody immunogen group comprising structural proteins of African swine fever virus; and
(B) A recombinant T-cell immunogen group comprising non-structural proteins of African swine fever virus.

In some embodiments, the antibody immunogen group comprises one or more full-length or extracellular fragments of structural proteins derived from African swine fever virus, for example, one or more proteins or fragments selected from the group consisting of B646L, B602L, H240R, B438L, C204L, D117L, E183L, and EP402R; or any combination thereof.

In some embodiments, the recombinant T-cell immunogen group comprises one or more T- cell epitopes comprised in non-structural proteins derived from African swine fever virus (ASFV) selected from the group consisting of F334L, H359L, D250R, D339L, F1055L, G1211R, A528, and MGF-505-7R proteins; or any combination of T-cell epitopes comprised in the aforesaid non-structural proteins; or fragments having at least 80% sequence identity to the aforementioned combinations of T-cell epitopes.

In some aspects of the present application, a recombinant T-cell immunogenic peptide is provided, comprising multiple peptide elements derived from non-structural proteins of African swine fever virus.

In some aspects of the present application, nucleic acid molecules encoding the immunogenic compositions or recombinant T-cell immunogenic peptides of the present application are provided. In some aspects of the present application, vectors or host cells comprising said nucleic acid molecules are provided.

In some aspects of the present application, an African swine fever vaccine is provided, comprising:

(I) One or more immunogenic components or precursors thereof selected from the group consisting of one or more immunogenic compositions, recombinant T-cell immunogenic peptides, nucleic acid molecules, vectors, and host cells of the present application; and
(II) One or more pharmaceutically or veterinarily acceptable carriers or excipients or delivery systems.

In some aspects of the present application, a product is provided comprising one or more of the immunogenic compositions, recombinant T-cell immunogenic peptides, nucleic acid molecules, vectors, host cells, or vaccines of the present application.

In some embodiments, the product is a vaccine kit comprising one or more doses of the same or different vaccines of the present application.

In some aspects of the present application, the use of one or more of the immunogenic compositions, recombinant T-cell immunogenic peptides, nucleic acid molecules, vectors or host cells, or vaccines of the present application in the preparation of products for the prevention and/or treatment of African swine fever is provided.

In some aspects of the present application, a method for preventing and/or treating African swine fever is provided, the method comprises administering to a subject in need thereof a prophylactically and/or therapeutically effective amount of the immunogenic composition, recombinant T-cell immunogenic peptide, nucleic acid molecule, vector or host cell, vaccine, or product of the present application.

Those skilled in the art may arbitrarily combine the above technical solutions and technical features without departing from the inventive concept and protection scope of the present invention. Other aspects of the present invention are obvious to those skilled in the art based on the disclosure of present application.

## DESCRIPTION OF FIGURES

The present invention will be further illustrated below in conjunction with the accompanying drawings, and these drawings are only for illustrating the embodiments of the present invention rather than limiting the scope of the present invention.

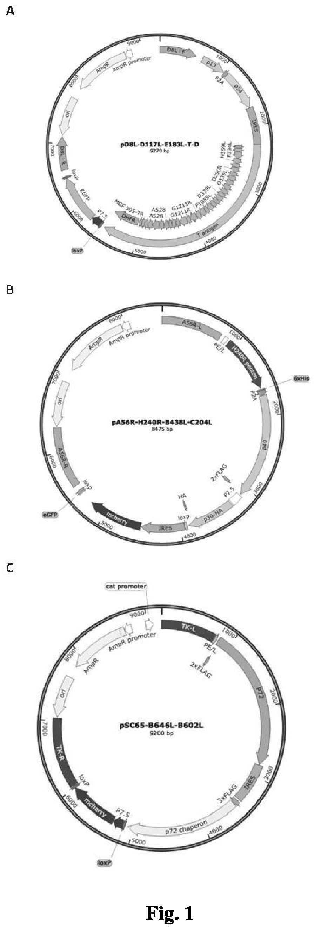

FIG. 1: **Maps of shuttle plasmids used in the examples:** A: pD8L-D117L-E183L-T-D (wherein T = T antigen, D = DHFR); B: pA56R-H240R-B438L-C204L; C: pSC65-B646L-B602L.

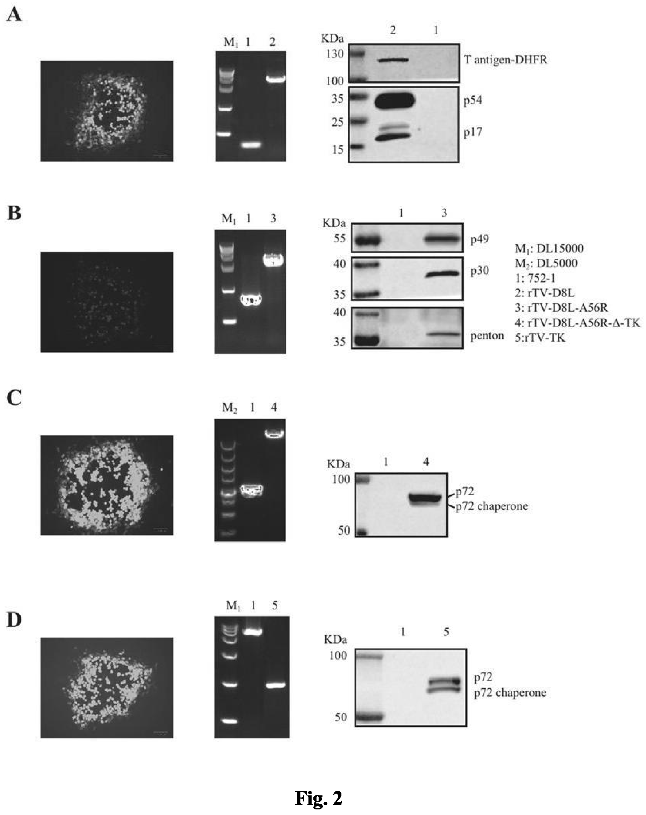

**FIG. 2: Construction, verification and expression of rTV-D8L-A56R-Δ-TK:** A: Left: schematic diagram of plaque isolation at the 5th round of rTV-D8L screening; Middle: PCR verification of rTV-D8L with D8L-F/D8L-R, showing the target band size; Right: expression of p17, p54 and T antigen by rTV-D8L. B: Left: schematic diagram of plaque isolation at the 7th round of rTV-D8L-A56R screening; Middle: PCR verification of rTV-D8L-A56R with A56-F/A56-R, showing the target band size; Right: expression of penton, p49 and p30 by rTV-D8L-A56R. C: Left: schematic diagram of plaque isolation at the 10th round of rTV-D8L-A56R-Δ-TK screening; Middle: PCR verification of rTV-D8L-A56R-Δ-TK with TK-F/TK-R, showing the target band size; Right: expression of p72 and p72 chaperone by rTV-D8L-A56-Δ-TK. D: Left: schematic diagram of plaque isolation at the 6th round of rTV-TK screening; Middle: PCR verification of rTV-TK with TK-F/TK-R, showing the target band size; Right: expression of p72 and p72 chaperone by rTV-TK.

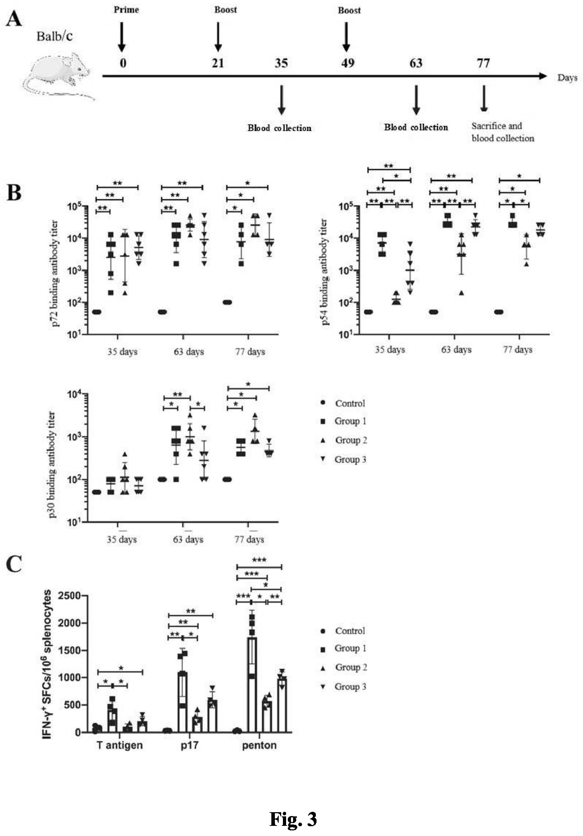

**FIG. 3: Immune response levels induced by different recombinant vaccinia virus combinations in mice:** A: schematic diagram of immunization procedure and time points; B: binding antibody titers against p72, p54 and p30 in mice of each group on Day 35, 63 and 77 post-immunization; C: cellular immune response levels against T antigen, p17 and penton induced in mice of each group.

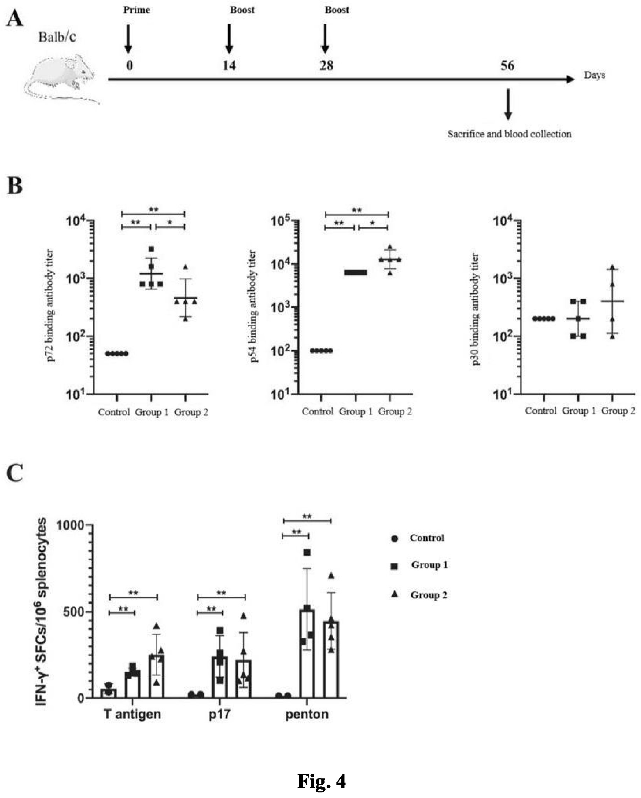

**FIG. 4: Immune response levels induced by other recombinant vaccinia virus combinations in mice:** A: schematic diagram of immunization procedure and time points; B: binding antibody titers against p72, p54 and p30 in mice of each group on Day 56 post-immunization; C: cellular immune response levels against T antigen, p17 and penton induced in mice of each group.

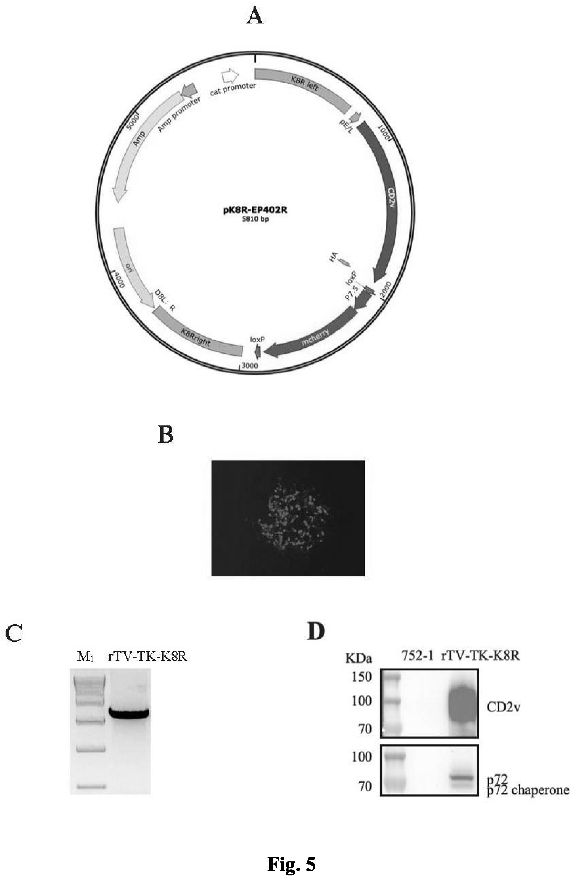

**FIG. 5: Construction, verification and expression of rTV-TK-K8R:** A: pK8REP402R shuttle plasmid map; B: schematic diagram of plaque isolation at the 9th round of rTV-TK-K8R screening; C: PCR verification of rTV-TK-K8R with K8R-F/K8R-R, showing the target band size; D: expression of CD2v by rTV-TK-K8R.

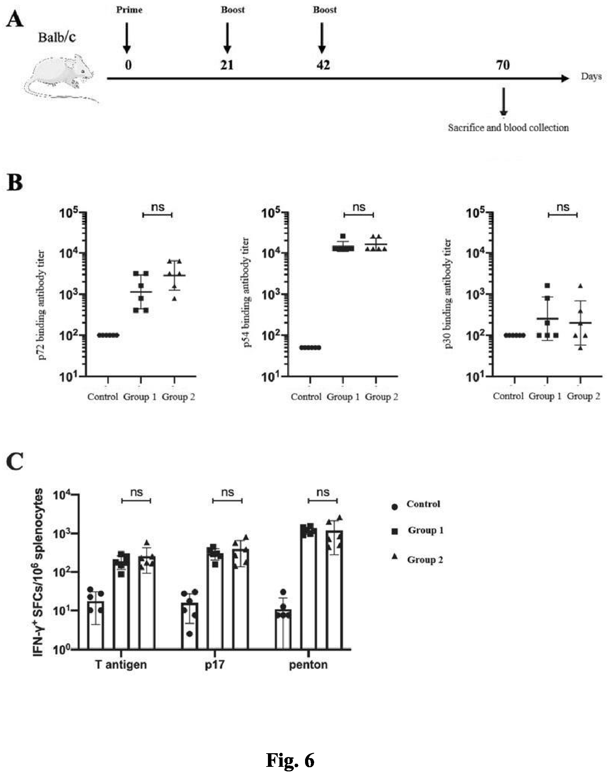

**FIG. 6: Immune response levels induced by different recombinant vaccinia virus combinations in mice:** A: schematic diagram of immunization procedure and time points; B: binding antibody titers against p72, p54 and p30 in mice of each group on Day 70 post-immunization; C: cellular immune response levels against T antigen, p17 and penton induced in mice of each group.

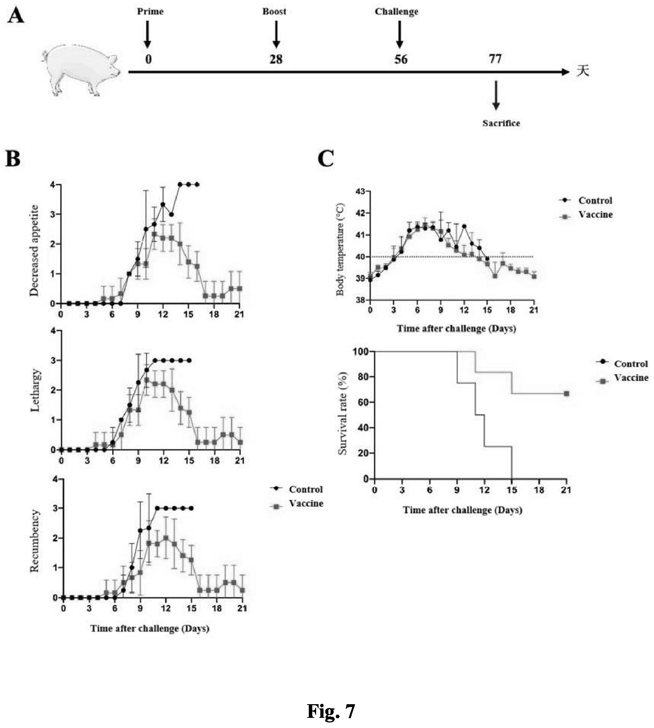

**FIG. 7: Protective effect of recombinant vaccinia virus combinations in field pigs *in vivo*:** A: schematic diagram of immunization procedure and time points; B (top): changes in clinical scores for feed intake status in the control group and immunized group post-challenge; B (middle): changes in clinical scores for mental status in the control group and immunized group post-challenge; B (bottom): changes in clinical scores for recumbency in the control group and immunized group post-challenge; C (top): changes in body temperature in the control group and immunized group post-challenge; C (bottom): survival curves of the control group and immunized group post-challenge.

In figures, * indicates $p < 0.05$, ** indicates $p < 0.01$, and *** indicates $p < 0.005$.

## DETAILED DESCRIPTION

The present disclosure relates to the field of vaccines, and particularly to a dual immunogen composition consisting of an antibody immunogen composed of structural proteins of African swine fever virus and a T-cell immunogen derived from non-structural proteins, a vaccine comprising the immunogen composition, and use thereof. In the present application, suitable African swine fever virus immunogens were selected, and conserved T-cell epitopes were loaded simultaneously. The above immunogens was then loaded using a appropriate vector, thereby developing a safe and effective African swine fever vaccine.

All numerical ranges provided herein are intended to clearly include all values falling between the range endpoints and the numerical ranges therebetween. Features mentioned in the present application or in the embodiments may be combined. All features disclosed in the present application may be used in combination with any composition form, and each feature disclosed in the present application may be replaced by any alternative feature that can provide the same, equal or similar purpose. Unless otherwise specified, the disclosed features are only general examples of equal or similar features.

As used therein, the term "about" in the context of a number or range means $\pm 10\%$ of the number or range referenced or claimed.

It should be understood that when a parameter range is provided, the present invention also provides all integers within that range and their tenths decimal places. For example,"0.1-2.5 mg/day" includes 0.1 mg/day,0.2 mg/day,0.3 mg/day, *etc.* ,up to 2.5 mg/day.

As used herein, "comprising", "having", or "including" encompasses "consisting of", "consisting essentially of", and "comprised of"; "consisting essentially of", "substantially consisting of" and "consisted of" are sub-concepts of "comprising", "having", or "including".

Immunogenic composition(s)

The present application provides an immunogenic composition comprising (A) An antibody immunogen group comprising immunogens derived from structural proteins of African swine fever virus; and (B) A recombinant T-cell immunogen group comprising immunogens derived from non-structural proteins of African swine fever virus.

In some embodiments, the immunogenic fragments in the immunogenic composition(s) of the present application are derived from African swine fever virus selected from circulating strains of African swine fever virus, and/or from multiple strains of African swine fever virus with different genotypes and serotypes.

In some embodiments, the African swine fever virus is one or more African swine fever virus strains selected from the group consisting of Pig/HLJ/18, China/Guangxi/2019/domestic pig, CN201801, China/Jilin/2018/boar, Pig/Hubei/628/2020, Pig/Liaoning/LC/2020, Pig/Hebei/Q3/2020, and Georgia 2007/1.

In some embodiments, the antibody immunogen group comprises one or more full-length or extracellular fragments of structural proteins derived from African swine fever virus.

In some embodiments, the antibody immunogen group comprises one or more proteins or fragments selected from the group consisting of B646L, B602L, H240R, B438L, C204L, D117L, E183L, and EP402R; or any combination thereof. In some embodiments, the combination is selected from a combination of D117L and E183L (D117L-E183L), a combination of H240R, B438L and C204L (H240R-B438L-C204L), a combination of B646L and B602L (B646L-B602L), a combination of D117L, E183L, H240R, B438L and C204L (D117L-E183L-H240R-B438L-C204L), a combination of D117L, E183L, H240R, B438L, C204L, B646L and B602L (D117L-E183L-H240R-B438L-C204L-B646L-B602L), and a combination of B646L, B602L, and EP402R (B646L-B602L -EP402R). In some embodiments, the proteins or fragments in any one of the combinations are directly linked or linked via one or more linkers, for example, a linker selected from SEQ ID NOs: 89-94, IRES, P2A, T2A, E2A, and F2A.

In some embodiments, B646L, B602L, H240R, B438L, C204L, D117L, E183L, and EP402R are peptide segments having the amino acid sequences set forth in SEQ ID NOs: 2, 4, 6, 8, 10, 12, 14, and 98, respectively, or peptide segments having at least 80% sequence identity or homology with the amino acid sequences set forth in SEQ ID NOs: 2, 4, 6, 8, 10, 12, 14, and 98.

In some embodiments, B646L, B602L, H240R, B438L, C204L, D117L, E183L, and EP402R are encoded by nucleic acid molecules having the nucleotide sequences set forth in SEQ ID NOs: 1, 3, 5, 7, 9, 11, 13, and 97, respectively, or by nucleic acid molecules having at least 80% sequence identity or homology with the nucleotide sequences set forth in SEQ ID NOs: 1, 3, 5, 7, 9, 11, 13, and 97.

In some embodiments, the recombinant T-cell immunogen group comprises one or more non-structural proteins or fragments thereof derived from African swine fever virus. In some embodiments, T-cell epitopes that are broadly recognized by porcine major histocompatibility complex (SLA) molecules are screened using bioinformatics and fused into T antigens, such as the T antigen set forth in SEQ ID NO: 78. In other embodiments, DHFR is inserted at the 3' end of the fused T antigen to further enhance the intracellular degradation level of the T antigen, such as the DHFR-comprising T antigen set forth in SEQ ID NO: 80.

In some embodiments, the recombinant T- cell immunogen group comprises (a) one or more T- cell epitopes comprised in non-structural proteins selected from the group consisting of F334L, H359L, D250R, D339L, F1055L, G1211R, A528, and MGF -505-7R proteins; or any combination of T- cell epitopes comprised in the aforementioned non-structural proteins; or fragments having at least 80% sequence identity with the aforementioned combination of T -cell epitopes; and (b) optionally, one or more linkers located between the peptide elements, for example, a linker selected from SEQ ID NOs: 89-94, IRES, P2A, T2A, E2A, and F2A.

In some embodiments, the recombinant T- cell immunogen group comprises at least a combination of T- cell epitopes comprised in F334L, H359L, D250R, D339L, F1055L, G1211R, A528, and MGF-505-7R proteins.

In some embodiments, the recombinant T- cell immunogen group comprises:

(i) At least 8, 10, 20, 30, or all peptide segments selected from the group consisting of peptide segments having the amino acid sequences set forth in SEQ ID NOs: 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, and 76, or peptide segments having at least 80% sequence identity with any one of the aforementioned peptide segments; and/or

(ii) A peptide segment having the amino acid sequence set forth in SEQ ID NO: 78 or 80, or a peptide segment having at least 80% sequence identity with any one of the aforementioned peptides; and/or

In some embodiments, the nucleic acid molecules encoding the recombinant T- cell immunogen group comprises:

(i') At least 8, 10, 20, 30 or all nucleic acid molecules selected from the group consisting of nucleic acid molecules having nucleotide sequences set forth in SEQ ID NOs: 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, and 75, or nucleic acid molecules having at least 80% sequence identity with any one of the aforementioned nucleic acid molecules; and/or (ii') nucleic acid molecules with nucleotide sequences set forth in SEQ ID NO: 77 or 79, or nucleic acid molecules having at least 80% sequence identity with any one of the aforementioned nucleic acid molecules.

In some embodiments, the recombinant T-cell immunogen group is linked or mixed with the antibody immunogen group, for example, the recombinant T-cell immunogen group is mixed or linked with B646L, B602L, H240R, B438L, C204L, D117L, E183L, EP402R, or any combination thereof (e.g., a combination of D117L and E183L), for example, each independently linked via one or more linkers selected from SEQ ID NOs: 89-94, IRES, P2A, T2A, E2A, and F2A.

Correspondingly, the present application also provides a recombinant T- cell immunogenic peptide, comprising:

(a) one or more T- cell epitopes comprised in non-structural proteins derived from African swine fever virus (ASFV) selected from the group consisting of F334L, H359L, D250R, D339L, F1055L, G1211R, A528, and MGF -505-7R proteins; or any combination of T-cell epitopes comprised in the aforesaid non-structural proteins; or fragments having at least 80% sequence identity with the aforementioned combinations of T-cell epitopes; and

(b) Optionally, one or more linkers located between the peptide elements, such as a linker selected from SEQ ID NOs: 89-94, IRES, P2A, T2A, E2A, and F2A.

In some embodiments, the recombinant T- cell immunogenic peptide comprises at least a combination of T -cell epitopes comprised in F334L, H359L, D250R, D339L, F1055L, G1211R, A528, and MGF-505-7R proteins.

In some embodiments, the recombinant T- cell immunogenic peptide comprises:

(i) At least 8, 10, 20, 30, or all peptide segments selected from the group consisting of peptide segments having the amino acid sequences set forth in SEQ ID NOs: 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, and 76, or peptide segments having at least 80% sequence identity with any one of the aforementioned peptide segments; and/or

(ii) A peptide segment having the amino acid sequence set forth in SEQ ID NO: 78 or 80, or a peptide segment having at least 80 % sequence identity with any of the aforementioned peptides.

In some embodiments, the nucleic acid molecule encoding the recombinant T- cell immunogenic peptide comprises:

(i') At least 8, 10, 20, 30, or all nucleic acid molecules selected from the group consisting of nucleic acid molecules having nucleotide sequences set forth in SEQ ID NOs: 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, and 75, or nucleic acid molecules having at least 80% sequence identity with any one of the aforementioned nucleic acid molecules; and/or

(ii') A nucleic acid molecule having the nucleotide sequence set forth in SEQ ID NO: 77 or 79, or a nucleic acid molecule having at least 80% sequence identity with any one of the aforementioned nucleic acid molecules.

The present application also provides nucleic acid molecules encoding the aforementioned immunogenic composition or recombinant T-cell immunogenic peptide.

Vector and host cell

The present application also provides vectors or host cells comprising the nucleic acid molecules described herein.

In some embodiments, the vector may be selected from mRNA vectors, DNA plasmid vectors (e.g., shuttle plasmid vectors), recombinant viral vectors, and recombinant bacterial vectors; wherein the mRNA vector is selected from linear, circular, and self-replicating vectors; and the recombinant viral vector is selected from vaccinia virus (e.g., Tiantan strain, North American vaccine strain, Wyeth derivative strain, Listeria strain, Ankara derivative strain, Copenhagen strain, and New York strain), adenovirus (e.g., adenovirus types 5, 11, 26, 35, 63, and 68), adeno-associated virus, herpes simplex virus, measles virus, enterovirus, reovirus, rhabdovirus, flavivirus, influenza virus, parainfluenza virus, respiratory syncytial virus, and poliovirus vectors.

In some embodiments, a vaccinia virus comprising immunogens of the present application or a combination thereof is provided. In some embodiments, an immunogen gene is inserted into one or more sites selected from the group consisting of D8L, A56R, TK, and K8R in the recombinant vaccinia virus (rTV) of the present application. In some embodiments, the recombinant vaccinia virus of the present application includes, but is not limited to, recombinant viruses that have inserted one or more of the following immunogenic fragments: D117L-E183L-T antigen, H240R-B438L-C204L, B646L-B602L, D117L-E183L-T antigen-H240R-B438L-C204L, D117L-E183L-T antigen-H240R-B438L-C204L-B646L-B602L, and B646L-B602L-EP402R.

In some embodiments, the recombinant vaccinia virus of the present application may be selected from the group consisting of rTV-D8L-D117L-E183L-T antigen, rTV-D8L-D117L-E183L-T antigen-A56R-H240R-B438L-C204L, rTV-D8L-D117L-E183L-T antigen-A56R-H240R-B438L-C204L-TK-B646L-B602L, rTV-TK-B646L-B602L, and rTV-TK-B646L-B602L-K8R-EP402R.

In some embodiments, the T antigen in the recombinant vaccinia virus is linked to DHFR to enhance the degradation level of the T antigen within cells. In some embodiments, the recombinant vaccinia virus is selected from the group consisting of rTV-D8L-D117L-E183L-T antigen-DHFR, rTV-D8L-D117L-E183L-T antigen-DHFR-A56R-H240R-B438L-C204L, rTV-D8L-D117L-E183L-T antigen-DHFR-A56R-H240R-B438L-C204L-TK-B646L-B602L, rTV-TK-B646L-B602L, and rTV-TK-B646L-B602L-K8R-EP402R.

In some embodiments, the vaccinia virus promoter is derived from a strong promoter that initiates viral protein expression in vaccinia virus, such as pE/L, p7.5, pH5, and different truncated forms of each strong promoter. Selection markers include reporter genes such as LacZ, eGFP, mcherry, BFP, and ZsGreen.

In some embodiments, the host cell may be a mammalian cell or an insect cell, such as HEK293, HeLa, K562, CHO, NSO, SP2/0, PER.C6, Vero, RD, BHK, HT 1080, A549, Cos-7, ARPE-19, and MRC-5 cells; High Five, Sf9, Se301, SeIZD2109, SeUCR1, Sf9, Sf900+, Sf21, BTI-TN-5B1-4, MG-1, Tn368, HzAm1, BM-N, Ha2302, Hz2E5, and Ao38.

Vaccines and products

The present application provides an African swine fever vaccine, comprising:

(I) One or more immunogenic components or precursors thereof selected from the group consisting of the immunogenic composition, recombinant T-cell immunogenic peptides, nucleic acid molecules, vectors, and host cells of the present application; and
(II) One or more pharmaceutically or veterinarily acceptable carriers or excipients or delivery systems.

In some embodiments, the African swine fever vaccine is an mRNA vaccine, a DNA vaccine, a viral vector vaccine (e.g., a vaccinia virus vaccine), or a recombinant protein vaccine.

In some embodiments, the carrier or excipient or delivery system is selected from lipid delivery systems, lipid-like delivery systems, polymer delivery systems or combinations thereof, such as loaded onto lipid nanoparticles (e.g., a combination of cationic lipids, structural lipids, auxiliary lipids and stabilizing lipids), polyurethane (PAA), poly-$\beta$-amino ester (PBAE), polyethyleneimine (PEI), and lipid-encapsulated polymer micelles.

In some embodiments, the vaccine also comprises or is used in combination with an adjuvant, for example, the adjuvant is selected from aluminum adjuvant, cholera toxin and subunits thereof, oligodeoxynucleotides, manganese ion adjuvant, colloidal manganese adjuvant, Freund's adjuvant, MF59 adjuvant, QS-21 adjuvant, Poly I:C and other TLR ligands, GM-CSF, IL-2, IL-3, IL-7, IL-11, IL-12, IL-18, and IL-21.

In some embodiments, the vaccine is in a form suitable for one or more administration or delivery methods selected from the group consisting of respiratory nebulization, nasal drops, oral administration, direct injection (e.g., intravenous injection, subcutaneous injection, intradermal injection, intramuscular injection), and mucosal administration.

In some embodiments, the vaccine is in a form suitable for administration of two or more drugs or vaccines in combination, such as combined or sequential administration. In some embodiments, the vaccine is a monovalent or multivalent vaccine.

In some embodiments, the vaccine is a recombinant vaccinia virus (rTV) vaccine. In some embodiments, the rTV vaccine comprises the following elements: a vaccinia virus vector; a vaccinia virus recombination site; a vaccinia virus shuttle plasmid; a vaccinia virus promoter; and a selection marker.

The application also provides a product for the prevention and/or treatment of African swine fever, comprising one or more of the immunogenic compositions, recombinant immunogenic peptides, nucleic acid molecules, vectors, or host cells or vaccines of the present application.

In some embodiments, the product is a vaccine kit comprising one or more doses of the same or different vaccines of the present application. In some embodiments, the product comprises one or more doses of a combination of recombinant vaccinia virus vaccines selected from the group consisting of rTV-D117L-E183L-T antigen, rTV-H240R-B438L-C204L, rTV-B646L-B602L, rTV-D117L-E183L-T antigen-H240R-B438L-C204L, rTV-D117L-E183L-T antigen-H240R-B438L-C204L-B646L-B602L, rTV-B646L-B602L, and rTV-B646L-B602L-EP402R.

In some embodiments, the product comprises one or more doses of a combination of recombinant vaccinia virus vaccines selected from the group consisting of any combination of rTV-D117L-E183L-T antigen-H240R-B438L-C204L and rTV-D117L-E183L-T antigen-H240R-B438L-C204L-B646L-B602L.

In some embodiments, the product comprises one or more doses of a combination of recombinant vaccinia virus vaccine selected from the group consisting of rTV-D117L-E183L-T antigen-H240R-B438L-C204L and rTV-D117L-E183L-T antigen-H240R-B438L-C204L-B646L-B602L, or rTV-B646L-B602L, or rTV-B646L-B602L-EP402R, or two or three doses of a combination of rTV-D117L-E183L-T antigen-H240R-B438L-C204L-B646L-B602L or rTV-B646L-B602L-EP402R.

In some embodiments, the product comprises one or more doses of a combination of recombinant vaccinia virus selected from the group consisting of rTV-D117L-E183L-T antigen-H240R-B438L-C204L and rTV-D117L-E183L-T antigen-H240R-B438L-C204L-B646L-B602L, or rTV-rTV-B646L-B602L, or rTV-B646L-B602L-EP402R; a combination of rTV-D117L-E183L-T antigen-H240R-B438L-C204L-B646L-B602L and rTV-B646L-B602L, or rTV-B646L-B602L-EP402R.

In some preferred embodiments of the present application, an African swine fever virus vaccinia virus vector vaccine is constructed by inserting an immunogen composition into a vaccinia virus vector. The recombinant vaccinia virus constructed comprises rTV-D8L (i.e., rTV-D8L-D117L-E183L-T antigen-DHFR), rTV-D8L-A56R (i.e., rTV-D8L-D117L-E183L-T antigen-DHFR-A56R-H240R-B438L-C204L), rTV-D8L-A56R-Δ-TK (i.e., rTV-D8L-D117L-E183L-T antigen-DHFR-A56R-H240R-B438L-C204L-TK-B646L-B602L), rTV -TK (i.e. rTV-TK-B646L-B602L), and rTV-TK-K8R (i.e. rTV-TK-B646L-B602L-EP402R).

Immunization method(s)

A method for preventing and/or treating African swine fever virus infection and/or its symptoms is also provided therein, the method comprises administering at least one dose of a prophylactically and/or therapeutically effective amount of one or more vaccines of the present disclosure. Possible routes of administration include, but are not limited to systemic immunization, such as intramuscular, subcutaneous, and intradermal injections; and intrarespiratory immunization, such as nebulization or nasal drops. In some embodiments, prime immunization is performed using systemic or intrarespiratory administration, with systemic administration being preferred.

In some embodiments of the present disclosure, the interval between two vaccinations is at least one week, such as two weeks, four weeks, two months, three months, six months or longer.

In some embodiments, the immunization method of the present disclosure may adopt a "prime-boost" or "prime-boost-reboost" regimen, and may employ a single systemic immunization or a respiratory local immunization approach, or a combination of two immunization approaches. In some embodiments, the scheme of prime immunization with rTV-D8L-A56R followed by two booster doses of rTV-D8L-A56R-Δ-TK can induce high titers of binding antibodies against p72, p54, and p30 *in vivo*, while simultaneously activate T-cell responses against T antigens, p17, and penton, thereby providing a prophylactical effect against African swine fever virus.

In some embodiments, prime immunization may use rTV-D8L-A56R, and one or two booster immunizations may be performed using a combination of rTV-D8L-A56R and rTV-TK or rTV-D8L-A56R-TK. In some embodiments, prime immunization may use rTV-D8L-A56R, and one or two booster immunizations may be performed using a combination of rTV-D8L-A56R and rTV-TK, or rTV-D8L-A56R-TK. In some embodiments, both prime and booster immunizations may use a combination of rTV-D8L-A56R and rTV-TK-K8R. In some embodiments, the interval between two vaccinations may be independently selected from 1 week, 2 weeks, 3 weeks, 4 weeks, 1 month, 2 months, 3 months, 6 months, or any interval within this range. For example, the interval between prime immunization and booster immunization can be 2, 3, or 4 weeks; the interval between booster immunization and reboost immunization can be 2, 3, or 4 weeks.

The combination products described herein may be provided in the form of drug packages or kits, for example, one or more vaccine compositions or one or more components thereof may be packaged in one or more containers, such as sealed containers, such as ampoules or capsules, specifying the amount of the composition. Vaccine compositions may be provided in the form of liquids, sterile lyophilized powders, or anhydrous concentrates, and may be diluted, reconstituted, and/or formulated with appropriate liquids (e.g., water, saline, etc.) to obtain the appropriate concentration and form for administration to the subjects prior to use.

The combined products disclosed herein can be used to simultaneously induce high levels of neutralizing antibodies and high levels of T-cell responses in subjects, providing dual protection and making them promising for broad application in the prevention and treatment of African swine fever virus infection.

**Examples**

The present disclosure will be further illustrated below with reference to specific example. It should be understood that these examples are provided for illustration only and are not intended to limit the scope of this application. Those skilled in the art may make appropriate modifications and changes to the present invention, and these modifications and changes are all within the scope of the present invention.

Experimental methods in the following examples, wherein specific conditions are not mentioned, can adopt conventional methods in the field, such as those referenced in "Molecular Cloning: A Laboratory Manual" (3rd edition, Cold Spring Harbor Laboratory Press, New York, 1989) or according to conditions recommended by suppliers. DNA sequencing methods are conventional in the field and can also be provided by commercial companies.

Unless otherwise stated, percentages and parts are calculated by weight. Unless otherwise defined, all technical and scientific terms used herein have the same meanings as those familiar to those skilled in the art. In addition, any methods and materials similar or equivalent to those described herein can be applied to the methods of the present invention. The preferred implementation methods and materials described herein are for demonstration purposes only.Specific information on the sequences used in the examples can be found in the appendix and sequence listing.

Experiments in examples involve the screening, amplification, *in vitro* purification, animal immunization protocols, and detection methods of African swine fever virus vaccinia virus vector vaccines, which are as follows.

## I. Screening of African swine fever virus vaccinia virus vector vaccines

### 1. Preparation of shuttle plasmids

Construction of shuttle plasmid pD8L-D117L-E183L-T-D comprising recombinant D8L site. The genome of vaccinia virus Tian Tan strain was extracted according to the instructions of the Vazyme Viral Genome Extraction Kit (Cat. No.: RC311-01). The genomic DNA was used as a template, approximately 700 bp fragments at the 5' and 3' ends of the D8L gene were amplified by PCR, respectively. According to the instructions of the Vazyme Homologous Recombination Kit (Cat. No.: C113-02), the original homologous arms of the vector plasmid pSC65 (purchased from BioVector NTCC Typical Culture Collection Center, Cat. No.: BioVector-790265) were replaced by homologous recombination, respectively. The D117L, E183L and T antigen-DHFR (T-D) genes were artificially synthesized; the D117 and E183 genes were linked by P2A, and the E183 and T antigen-DHFR genes were linked by IRES. The target gene D117L-P2A-E183L-IRES-T-D was amplified by PCR and ligated to the 3' end of the pE/L promoter on the pSC65 vector. The p7.5 promoter and downstream eGFP gene were inserted at the 3' end of the target gene, and loxp sequences (SEQ ID NO: 82) were inserted at both ends of the p7.5-eGFP gene. Finally, the shuttle plasmid pD8L-pE/L-D117L-P2A- E183L-IRES-TD -loxp-p7.5-eGFP-loxp (hereinafter referred to as pD8L-D117L-E183L-T- D) was constructed.

Using substantially the same method as above, the corresponding target genes were artificially synthesized and inserted according to the plasmid maps shown in Figs. 1B, 1C, and 6A, respectively, to prepare the following shuttle plasmids: pA56R-H240R-B438L-C204L (pA56R-pE/L-H240R-P2A-B438L-p7.5-C204L-IRES-loxp-mcherry-loxp), pSC65-B646L-B602L (pSC65-pE/L-B646L-IRES-B602L-loxp-p7.5-eGFP-loxp) and pK8REP402R.

### 2. Shuttle plasmid for recombinant wild-type vaccinia virus

Construction of recombinant vaccinia virus rTV-D117L-P2A-E183L-IRES-T-D comprising recombinant D8L site. Approximately $1 \times 10^6$ 293T cells (purchased from ATCC, Cat. No.: CRL-3216) were seeded in a single well of a 6-well plate one day in advance, ensuring a cell density of 80% by the next day. The cells were infected with wild-type vaccinia virus 752-1 (purchased from China Center for Type Culture Collection) at an MOI of 0.02. Two hours after infection, the complete culture medium comprising viral supernatant was discarded, 2 mL of fresh maintenance medium was added, and the pD8L-D117L-E183L-T-D plasmid was transfected (transfection reagent purchased from Liji Biotechnology). The plate was incubated in an incubator at 37 °C with 5% $CO_2$ for approximately 24 h. Cells were collected the next day and freeze-thawed three times at -80 °C.

Using substantially the same method as above, recombinant poxviruses recombined with pA56R-H240R-B438L-C204L, pSC65-B646L-B602L or pK8REP402R were generated.

### 3. Screening of recombinant vaccinia virus

Approximately $8 \times 10^5$ 143B cells (purchased from ATCC, Cat. No.: CRL-8303) were seeded into each well of a 6-well plate one day in advance, ensuring a cell density of 95% by the next day. The complete culture medium in the wells was discarded, 2 mL of fresh maintenance medium was added, and each well was infected with 10 μL of the above freeze-thawed cells. The plate was then incubated in an incubator at 37 °C with 5% $CO_2$ for approximately 24 h. The culture medium in the wells was discarded on the next day, 2% agarose and 2×DMEM medium were mixed at a ratio of 1:1, and 1 mL was added to each well. The plate was allowed to stand at 4 °C for approximately 5 min, and then fluorescent plaques were selected under a fluorescence microscope. The plaques were aspirated with a 1 mL pipette tip into a 1.5 mL EP tube containing 500 μL maintenance medium, and freeze-thawed three times at -80 °C. The above step was repeated for approximately 6-8 rounds until the recombinant vaccinia virus was successfully screened. The genomic DNA thereof was extracted and subjected to PCR verification using primers with homologous arms at both ends. There should only be fragments that match the size of the target gene, and no wild-type virus fragments.

**II. Amplification and purification of African swine fever virus vaccinia virus vector vaccine**

**1. Amplification of recombinant vaccinia virus**

Successfully screened recombinant vaccinia virus was amplified in six-well plates until all cells were infected. Cells were collected and freeze-thawed three times at -80 °C.Approximately $1\times10^7$ DF-1 cells (purchased from ATCC, Cat. No.: CRL-3586) were seeded into 10 cm petri dishes one day in advance, ensuring a cell density of 95% by the next day. The complete culture medium in the petri dishes was discarded, and 10 mL of fresh maintenance medium was added. The cells were then infected with freeze-thawed virus and incubated in an incubator at 37 °C with 5% $CO_2$. Infected cells were collected within 24-48 hours based on cytopathic effects and freeze-thawed three times at -80 °C. Approximately $3\times10^7$ DF-1 cells were seeded in 15 cm petri dishes one day in advance, ensuring a cell density of 95% by the next day. The above steps were repeated, and the infected cells were collected and freeze-thawed three times at -80 °C. DF-1 cells were expanded into T175 culture flasks, passaged at a ratio of 1:10 after reaching confluence, and the remaining cells were plated in 15 cm petri dishes at a ratio of 1:10. After 48 h, the culture medium in the petri dishes was replaced with 20 mL maintenance medium. The seeds collected in the previous round were used for infection at a ratio of 1:10 (i.e., cells from one 15 cm petri dish were used to infect ten(10) 15 cm dishes). The infected cells were collected and freeze-thawed three times at -80 °C. DF-1 cells were expanded into six(6) T175 culture flasks. The above steps were repeated, the remaining cells were plated in sixty(60) 15 cm petridishes at a ratio of 1:10, and infected with the seeds collected in the previous round at a ratio of 1:10. The infected cells were collected and freeze-thawed three times at -80 °C. The step was repeated 2-3 rounds depending on the required amount of virus. Cells from each round of infection were collected and stored at -80 °C.

**2. Purification and titer determination of recombinant vaccinia virus**

The collected cells were freeze-thawed three times at -80 °C, then centrifuged at 3000g for 10 min, and the supernatant was collected. The pellet was resuspended in 10 mL of PBS, centrifuged at 3000g for 10 min, and the supernatant was collected. The washing step was repeated three times. 12 mL of 36% sucrose was added to an ultracentrifuge tube, the tube was tilted, and 24 mL of viral supernatant was slowly added at a ratio of 1:2 to ensure a clear visible stratification. The tubes were weighed using an electronic balance, and the balance was maintained with an error within 0.1 g. The ultracentrifuge parameters were set as follows: rotational speed 18000 g, temperature 4 °C, acceleration 9, deceleration 4, time 1.5 h. After centrifugation, a distinct white precipitate was visible at the bottom of the centrifuge tube. The supernatant was carefully discarded, excess supernatant was removed with a pipette tip, the precipitate was resuspended in 200 $\mu$L PBS and collected, and the precipitate was pipetted three times with 100 $\mu$L PBS and collected. The precipitate was aliquoted at 100 $\mu$L per tube, and 5 $\mu$L was separately aliquoted for titer determination and stored at -80 °C.

Approximately $2\times10^5$ 143B cells were seeded in each well of a 24-well plate one day in advance, ensuring a cell density of 95% by the next day. Eight 1.5 mL EP tubes were prepared, 396 $\mu$L of maintenance medium was added to the first EP tube, and 990 $\mu$L of maintenance medium was added to each of the remaining tubes. 5 $\mu$L aliquoted virus was taken out from the -80 °C fridge, 4 $\mu$L was aspirated and added to the first EP tube for 1:100 dilution, and after thorough pipetting and mixing, 110 $\mu$L was aspirated and added to the second EP tube for 1:10 dilution. Serial 10-fold dilutions were performed up to the last EP tube. The culture medium in the 24-well plate was discarded, each dilution factor for the culture medium was thoroughly mixed and added to two wells, 450 $\mu$L per well, in descending order of dilution factor. The plate was incubated in an incubator at 37 °C with 5% $CO_2$. After 48 hours, the number of plaques at the endpoint of the dilution factor was counted, and the viral titer was calculated according to the following formula:

Viral titer = Sum of plaque counts in two wells at the same dilution factor $\times$ $10^{\text{dilution factor}}$ (PFU/mL)

**III. Mouse Immunization Protocol**

**1. Experimental animals**

Female BALB/c mice aged 6 to 8 weeks were purchased from Shanghai Jihui Experimental Animal Breeding Co., Ltd.

**2. Route of immunization**

Intramuscular injection was administered to the lateral side of the left and right hind limbs of the mice.

### 3. Immunogen preparation and immunization dosage

The preparation of the immunogen was performed as described in Examples 1 and 4.

The immunization dosage was 1E7 PFU/ mouse, 100 $\mu$L.

### 4. Immunization interval

In Example 2, a prime immunization was performed on Day 0, followed by a booster immunization on Day 21 and a second booster on Day 49. In Example 3, a prime immunization was performed on Day 0, followed by a booster on Day 14 and a second booster on Day 28. In Example 5, a prime immunization was performed on Day 0, followed by a booster on Day 21 and a second booster on Day 42.

### IV. Mouse detection protocol

### 1. Blood collection

Blood was collected from the orbital venous plexus of mice on Day 35, 63, and 77, respectively, and placed into 1.5 mL EP tubes. The blood samples were allowed to stand at room temperature until blood coagulation. After centrifugation at 7000 g for 15 min, serum was transferred to a clean 1.5 mL EP tube, centrifuged at 14000 g for 2 min, and transferred to another clean 1.5 mL EP tube. The serum was inactivated in a 56 °C water bath for 30 min.

### 2. ELISA detection of binding antibodies

1) Antigen proteins (p72, purchased from Sino Biological Inc.; p54 and p30 purchased from Antibody System) were diluted to a final concentration of 1 $\mu$g/mL in ELISA dilution buffer, and 100 $\mu$L per well was added for coating overnight.

2) On the following day, the liquid in the wells was discarded, and the wells were washed three times with PBST containing 0.05% Tween-20, 200 $\mu$L per well per wash. After the last wash, residual liquid in the wells was removed by tapping dry thoroughly.

3) Each well was blocked with 200 $\mu$L of blocking buffer (5% skim milk in PBST) at room temperature for 2 hours.

4) After blocking, the liquid in the wells was discarded, and the wells were washed three times with PBST containing 0.05% Tween-20, 200 $\mu$L per well per wash. After the last wash, residual liquid in the wells was removed by tapping dry thoroughly.

5) Except for the first well, 100 $\mu$L of blocking buffer was added to each of the remaining sample wells. Serum samples were diluted 100-fold with blocking buffer, mixed thoroughly, and 200 $\mu$L was added to the first well of each sample. Two-fold serial dilutions were then performed by aspirating 100 $\mu$L to the next dilution well, mixed thoroughly, and the process was repeated. After the last sample well is thoroughly mixed, 100 $\mu$L of liquid was discarded, and incubated at room temperature for 3 hours.

6) The liquid in the wells was discarded, and the wells were washed 5 times with PBST containing 0.05% Tween-20, 200 $\mu$L per well per wash. After the last wash, residual liquid in the wells was removed by tapping dry thoroughly.

7) HRP-conjugated secondary antibody (goat anti-mouse, purchased from Yeasen) was diluted 1:5000 in blocking buffer, and 100 $\mu$L was added per well and incubated at room temperature for 1 hour.

8) The liquid in the wells was discarded, and the wells were washed 5 times with PBST containing 0.05% Tween-20, 200 $\mu$L per well per wash. After the last wash, residual liquid in the wells was removed by tapping dry thoroughly.

9) each pair of gold and silver OPD substrate tablets was dissolved in 20 mL of deionized water, 100 $\mu$L was added to each well, and the reaction was carried out in the dark for 5 min.

10) The reaction was stopped by adding 50 $\mu$L of 2M $H_2SO_4$ per well, and OD values at 492 nm and 630 nm were measured using a microplate reader.

11) The cut-off value was defined as twice the $OD_{492}$ value of the negative control. The reciprocal of the last dilution factor that exceeded the cut-off value was defined as the antibody titer.

### 3. ELISpot detection of T cell response

Isolation of mouse spleen single cells.

1) Mice were sacrificed and placed in lateral recumbency with the left side upward. The left abdominal skin was incised, the peritoneum was opened, and the spleen was harvested with sterile forceps and placed in a 6 cm petri dish containing 5 mL of complete RPMI 1640 medium (R10).
2) The spleen was wrapped with sterile gauze, the gauze was clamped with curved forceps, and the spleen was gently ground to release splenocytes into the culture medium.
3) The splenocyte suspension was transferred to a 15 mL centrifuge tube and centrifuged at 800 g for 5 min.
4) The supernatant was discarded, the cell pellet was resuspended by gentle tapping, 5 mL of red blood cell lysis buffer was added to each tube, mixed by inversion, and was allowed to stand at room temperature for 5 min.
5) 8 mL of R10 was added to terminate lysis, followed by centrifugation at 800 g for 5 min.
6) The supernatant was discarded, and the cells were washed once with 5 mL of R10, then centrifuged at 800 g for 5 min.
7) The supernatant was discarded, the cells were resuspended in 5 mL of R10, 20 $\mu$L of cell suspension was diluted 20-fold in 380 $\mu$L of R10, and the required volume of cell suspension according to the experimental design was aspirated after counting.
8) The remaining cells were centrifuged, the supernatant was discarded, and the cells were cryopreserved in freezing medium (90% FBS + 10% DMSO) for later use.

ELISpot assay was performed according to the instructions of the MOUSE IFN-$\gamma$ kit (BD, Cat. No. 551083).

1) IFN-$\gamma$ antibody was diluted 1:200 with sterile PBS, 100 $\mu$L was added to each well of a Millipore plate, and coated overnight at 4 °C.
2) The liquid in the plate was discarded, the plate was washed once with 200 $\mu$L of R10 and was allowed to stand for 3 min, and the liquid was then removed. 200 $\mu$L of fresh R10 was added, and blocked at room temperature for 2 h.
3) The liquid in the plate was discarded, and according to the experimental design, stimulating peptide pools were added at 50 $\mu$L to each well, with each single peptide having a working concentration of 5 $\mu$g/mL. 50 $\mu$L of R10 was added to each well for the negative control.
The stimulating peptide pools were synthesized by Suzhou Qiangyao Biotechnology Co., Ltd., covering T antigen, p17, and penton sequences, respectively. Each peptide pool had 10 single peptides, wherein:

- The T antigen comprised 31 T cell epitopes (polypeptides as set forth in SEQ ID NOs: 14+2n, wherein n=1-31), with each individual peptide consisting of 15 amino acids. The first peptide of the first epitope was FMREIKVKEVLYFYT, corresponding to aa 1-15 of SEQ ID NO: 16; and each subsequent peptide was shifted by 4 amino acids toward the C-terminus relative to the preceding peptide. The first peptide of the second epitope was GRVPGFSKEMLFQYI, corresponding to aa 1-15 of SEQ ID NO: 18; and each subsequent peptide was shifted by 4 amino acids toward the C-terminus relative to the preceding peptide, and so forth for each epitope. This pattern was applied to each epitope so as to cover all epitopes of the T antigen.
- The first peptide of p17 was MDTETSPLLSHNLST, corresponding to aa 1-15 of SEQ ID NO: 12, and each subsequent peptide was shifted by 4 amino acids toward the C-terminus relative to the preceding peptide. Accordingly, the second peptide corresponded to aa 5-19 of SEQ ID NO: 12, and the third peptide corresponded to aa 9-23 of SEQ ID NO: 12, and so forth. This pattern was continued until the twenty-seventh peptide, IPSDEQLAELAHS, corresponding to aa 105-117 of SEQ ID NO: 12, which comprised fewer than 15 amino acids. To maintain a peptide length of 15 amino acids, a twentyeighth peptide, SHIPSDEQLAELAHS, corresponding to aa 103-117 of SEQ ID NO: 12, was synthesized.
- The first peptide of penton was MAANIIATRAVPKMA, corresponding to aa 1-15 of SEQ ID NO: 6, and each subsequent peptide was shifted by 4 amino acids toward the C-terminus relative to the preceding peptide. Accordingly, the second peptide corresponded to aa 5-19 of SEQ ID NO: 6, and the third peptide corresponded to aa 9-23 of SEQ ID NO: 6, and so forth. This pattern was continued until the fifty-eighth peptide, RIPLYFKSLKTSK, corresponding to aa 229-241 of SEQ ID NO: 6, which comprised fewer than 15 amino acids. To maintain a peptide length of 15 amino acids, a fifty-ninth peptide, RIRIPLYFKSLKTSK, corresponding to aa 227-241 of SEQ ID NO: 15, was synthesized.

4) Mouse splenocytes were counted and adjusted to $4\times10^6$ cells/mL, and 50 $\mu$L ($2\times10^5$ cells per well) was added vertically to each well. The Millipore plate was incubated in an incubator at 37 °C with 5% $CO_2$ for 20 h without movement.

5) After incubation, the Millipore plate was taken out, the liquid was discarded, and 200 $\mu$L of pre-chilled distilled water was add to each well for wash twice, 5 min each time.

6) The liquid was discarded, 200 $\mu$L of PBST containing 0.05% Tween-20 was added to each well to wash 3 times, 1 min each time. After the last wash, the remaining liquid was patted dry on absorbent paper.

7) Biotin-conjugated detection antibody was diluted 1:250 in antibody dilution buffer (PBS + 10% FBS), 100 $\mu$L was added per well, and incubated at room temperature for 2 h.

8) After incubation, the liquid was discarded, 200 $\mu$L of PBST containing 0.05% Tween-20 was added to each well to wash 3 times, 1 min each time. After the last wash, the remaining liquid was patted dry on absorbent paper.

9) Streptavidin-HRP was diluted 1:100 in antibody dilution buffer (PBS + 10% FBS), 100 $\mu$L was added per well, and incubated at room temperature for 1 h.

10) The liquid was discarded, and 200 $\mu$L of PBST containing 0.05% Tween-20 was added to each well to wash 4 times.

11) 200 $\mu$L of PBS was added to wash twice. After the last wash, the remaining liquid was patted dry on absorbent paper.

12) Color development solution was prepared immediately before use, wherein 1 drop of color development substrate was added to each 1 mL of color development buffer, mixed well, and added at 100 $\mu$L per well. The reaction was conducted in the dark at room temperature for 5-60 min.

13) The degree of color development was carefully observed, and when a clear red spots appeared, the plate was gently rinsed under tap water for 5 minutes to stop the reaction.

14) After air-drying at room temperature, the plate was read and counted. Spot forming cells (SFCs) were counted and QC analyzed using a ChampSpot III enzyme-linked spot image analyzer.

**V. Challenge protection evaluation protocol for experimental pigs**

**1. Experimental animals**

40-50-day-old field pigs, purchased from Harbin Veterinary Research Institute.

**2. Route of immunization**

Intramuscular injection in the triangular region posterior to the pig ear.

**3. Immunogen preparation and immunization dosage**

The preparation of the immunogen was performed as described in Examples 1 and 5.

The immunization dosage was $2\times10^8$-$5\times10^8$ PFU per pig, 1 mL.

**4. Immunization interval**

Prime immunization was performed on Day 0 of the experiment, and booster immunization was administered 28 days later.

**5. Virus strain challenged:** HLJ/18

**6. Challenge procedure**

Challenge was conducted in the P4 Laboratory of Harbin Veterinary Research Institute. Challenge was performed on Day 28 after the completion of immunization, with each pig intramuscularly injected with 100 $HAD_{50}$ of African swine fever virus. Continuous observation was carried out for 21 days post-challenge. Body temperature was measured daily, and clinical scores and survival were recorded.

**Example 1. Construction, amplification, purification and expression validation of recombinant vaccinia virus**

In order to construct a safe and effective African swine fever vaccine, we reviewed literature and summarized information to select highly immunogenic exogenous genes. To further activate the cellular immune response in pigs, we used bioinformatics to select T cell epitopes that are broadly recognized by the porcine major histocompatibility complex (SLA) molecules and fused them into the T antigen (SEQ ID NO: 78). A DHFR (SEQ ID NO: 80) sequence was inserted at the 3' end to enhance the degradation level of the T antigen within cells. Subsequently, we artificially synthesized eight exogenous genes, including B646L, B602L, H240R, B438L, C204L, D117L, E183L, and T antigen, and inserted them into a vaccinia virus vector to construct an African swine fever virus vaccinia virus vector vaccine.

Eight exogenous genes were divided into three groups: ① D117L, E183L, and T antigen; ② H240R, B438L, and C204L; ③ B646L and B602L. It is important to note that this grouping method is only one possible combination, and other combinations that can achieve expression of all exogenous genes are within the scope of the present patent. Following Experimental Method I, shuttle plasmids for the D8L, A56R, and TK regions were constructed respectively. The three groups of exogenous genes were sequentially inserted into these three shuttle plasmids, resulting in the shuttle plasmid maps shown in Fig.1.

Following Experimental Method I, the shuttle plasmid pD8L-D117L-E183L-Tantigen DHFR was recombined with 752-1. Recombinant vaccinia virus was screened using eGFP (Fig.2A, left panel). After five rounds of screening, rTV-D8L-D117L-E183L-T antigen-DHFR (hereinafter referred to as rTV-D8L) was obtained. After small-scale amplification of rTV-D8L in a six-well plate, the genome was extracted and used as a template for PCR verification using primers D8L-F/D8L-R (SEQ ID NOs: 83 and 84). As shown in Fig.2A, the PCR showed only a single band, with a size consistent with the expected 6223 bp.

The expression of rTV-D8L was further detected, and approximately $8\times10^5$ 143B cells were seeded into each well of a six-well plate one day prior to infection to ensure a cell density of 95% the following day. The culture medium was replaced with 2 mL of maintenance medium, and rTV-D8L was used to infect the cells at an MOI of 0.2 in a well, while another well was left untreated. After 24 hours, the cells were collected and processed for Western blot analysis.As shown in Fig. 2A (right panel), a substantial expression of p17 and p54 proteins was detected after infection with rTV-D8L, while the T antigen showed relatively lower expression levels due to the inclusion of DHFR.Next, based on rTV-D8L, the shuttle plasmid pA56R-H240R-B438L-C204L, which harbors the mCherry reporter gene, was recombined to generate a dual-recombinant vaccinia virus, selected via mcherry screening (Fig. 2B, left panel). After seven rounds of selection, the recombinant virus rTV-D8L-D117L-E183L-T antigen-DHFR-A56R-H240R-B438L-C204L (hereinafter referred to as rTV-D8L-A56R) was obtained. The rTV-D8L-A56R was expanded in a six-well plate, and the viral genome was extracted as a template for PCR verification using primers D8L-F/D8L-R and A56R-F/A56R-R (SEQ ID NOs: 85 and 86). The PCR results showed a stable single band for the D8L region and a single band for the A56R region, with a size corresponding to the expected 4925 bp (Fig. 2B). The expression of rTV-D8L-A56R was further detected by Western blot.In addition to expressing the originally inserted p17, p54, and T antigens, rTV-D8L-A56R also highly expressed penton, p49, and p30, with sizes consistent with expectations (Fig.2B, right panel only shows the expression of penton, p49, and p30).

As the eGFP selection marker was incorporated in the pSC65-B646L-B602L, before inserting B646L-B602L into rTV-D8L-A56R, the eGFP and mCherry reporter genes in rTV-D8L-A56R were knocked out using the cre-loxp system. Approximately $1\times10^6$ 293T cells were seeded into each well of six-well plate one day prior to transfection, ensuring a cell density of approximately 80% the following day. The cells were transfected with 4 μg of phage-cre plasmid (SEQ ID NO: 95) from Liji for 6 hours, followed by infection with rTV-D8L-A56R at an MOI of 0.02. After 2 hours, the supernatant was discarded, and 2mL of fresh maintenance medium was added. Depending on the cell infection status, the cells were collected approximately 24 hours later and subjected to three freeze-thaw cycles at -80 °C.Following Experimental Method I, vaccinia virus strains not expressing eGFP and mCherry were reversely screened using fluorescence microscopy. After three rounds of screening, rTV-D8L-A56R-eGFPΔ-mCherryΔ (hereinafter referred to as rTV-D8L-A56R-Δ) was obtained. Using the extracted genome as a template, PCR verification was performed using primers D8L-F/D8L-R and A56R-F/A56R-R. A single band was observed at the target location, and protein immunoblotting detection confirmed the expression of the target gene. Next, the shuttle plasmid pSC65-B646L-B602L was recombined based on rTV-D8L-A56R-Δ. After 10 rounds of screening, rTV-D8L-D117L-E183L-T antigen-DHFR-A56R-H240R-B438L-C204L-TK-B646L-B602L (hereinafter referred to as rTV-D8L-A56R- Δ -TK) was obtained (Fig.2C, left panel). Genome were extracted and used as templates, PCR verification with primers D8L-F/D8L-R (SEQ ID NOs: 83 and 84), A56R-F/A56R-R (SEQ ID NOs: 85 and 86), and TK-F/TK-R (SEQ ID NOs: 87 and 88) showed stable bands for the D8L and A56R regions, and a single band for the TK region corresponding to the expected 6331 bp size (Fig. 2C). Western blot analysis detected the expression of p17, p54, Tantigen, penton, p49, p30, and p72, as well as p72 chaperone (Fig. 2C, right panel shows only the expression of p72 and p72 chaperone).

As described in Experimental Method I, the shuttle plasmid pSC65-B646L-B602L was recombined with 752-1, and the recombinant vaccinia virus was screened by eGFP (Fig.2D, left panel). After screening, rTV-TK-B646L-B602L (hereinafter referred to as rTV-TK) was obtained. The insertion and expression of the exogenous gene were confirmed to be correct by PCR and protein immunoblotting (Fig.2D, middle and right panels).

The above data demonstrated the successful screen and construction of rTV-D8L-A56R, rTV-D8L-A56R-Δ-TK, and rTV-TK, with correct insertion of the target genes into different recombination sites and normal expression.

**Example 2. Different combinations of recombinant vaccinia virus induce immune responses in mice *in vivo*.**

The selected rTV-D8L-A56R, rTV-D8L-A56R-Δ-TK, and rTV-TK were extensively amplified in large quantities according to Experimental Method I. After viral purification, the titers were measured to be $5 \times 10^8$ PFU/mL, $8 \times 10^8$ PFU /mL, and $1.1 \times 10^9$ PFU/mL, respectively. Twenty-four BALB/c mice were randomly divided into four groups and immunized according to Table 1. All groups received intramuscular injections, with a single immunization volume of 100 μL per mouse and a dose of 1E7 PFU. The immunization procedure is shown in Fig.3A. Peripheral blood from the orbital venous plexus of mice was collected on Day 35, 63, and 77. Enzyme-linked immunosorbent assay (ELISA) was used to evaluate the levels of binding antibodies against p72, p54, and p30 in the mice. On day 77, mouse spleen cells were isolated, and ELISpot was used to analyze the specific T cells induced by different recombinant vaccinia virus combinations against T antigen, p17, and penton.

As shown in Fig. 3B, the levels of binding antibodies against p72, p54, and p30 significantly increased with the number of immunizations. Compared to the two-dose immunization, after three doses, the antibody levels increased dramatically at the same time points (Day 35 vs. Day 63), with the highest fold increase for p72, p54, and p30 being 9-fold, 25.4-fold, and 9-fold, respectively. Compared to Day 63, the antibody levels on Day 77 remained generally stable or slightly increased. There was no significant difference in the induced anti-p72 binding antibody levels between different immunization combinations, with antibody titers on Day 77 being 7611, 25600, and 9051, respectively. Group 1 had a significantly higher anti-p54 antibody titer (7184) on day 35 compared to the other two groups. On Day 77, the antibody titers for p54 in Groups 1 and 3 were not significantly different, whereas the three-dose rTV-D8L-A56R-Δ-TK regimen resulted in a p54 antibody titer of 5382, indicating an imbalance in the induction of binding antibodies. The induced anti-p30 binding antibody titers were generally low across all groups, likely due to the weaker promoter activity of the p7.5 promoter driving p30 expression, as compared to the pE/L promoter. Consequently, p30 expression levels were lower in mice than p72 and p54. On day 77, Group 3 showed a p30 antibody titer of 1345, while the titers for other groups ranged between 400 and 600.

The results of the T-cell immune response are shown in Fig.3C. Compared with the control group, recombinant vaccinia virus induced cellular immune responses against T antigen, p17, and penton in mice. Group 1 showed the best induction results, followed by Group 3, and then Group 2. The T-cell response against the T antigen peptide library was weaker than the responses against the p17 and penton peptide libraries. This may be because the T antigen immunogen was screened by assessing its binding to porcine SLA molecules, which differs from mouse MHC molecules in species diversity.

These data suggest that a prime immunization with rTV-D8L-A56R, followed by two booster doses of rTV-D8L-A56R-Δ-TK, induced higher levels of anti-p72, anti-p54, and anti-p30 antibodies and T cell responses in mice. The three-dose rTV-D8L-A56R + rTV-TK combination was superior to the three-dose rTV-D8L-A56R-Δ-TK regimen, inducing antibody levels comparable to Group 1.

**Table 1. Immunogenicity of African swine fever virus vaccinia virus vector vaccine in BALB/c mice *in vivo*.**

|  | 0 days | 21 days | 49 days |
|---|---|---|---|
| Control group (n=6) | 752-1 | 752-1 | 752-1 |
| Group 1 (n=6) | rTV-D8L-A56R | rTV-D8L-A56R-Δ-TK | rTV-D8L-A56R-Δ-TK |
| Group 2 (n=6) | rTV-D8L-A56R-Δ-TK | rTV-D8L-A56R-Δ-TK | rTV-D8L-A56R-Δ-TK |
| Group 3 (n=6) | rTV-D8L-A56R +rTV-TK | rTV-D8L-A56R +rTV-TK | rTV-D8L-A56R +rTV-TK |

**Example 3. Optimization of recombinant vaccinia virus combination to induce immune response in mice *in vivo*.**

Based on the results of Example 2, we further explored better recombinant vaccinia virus combinations. Fifteen BALB/c mice were randomly divided into three groups and immunized according to the methods shown in Table 2, with the immunization procedure illustrated in Fig.4A. Mice were sacrificed on Day 56, and the levels of binding antibodies against p72, p54, and p30 in the mice were evaluated using an enzyme-linked immunosorbent assay (ELISA). Simultaneously, mouse spleen cells were isolated, and the specific T cell activity against T antigens, p17, and penton induced by different recombinant vaccinia virus combinations was analyzed using ELISpot.

**Table 2. Immunogenicity of African swine fever virus vaccinia virus vector vaccine combination in BALB/c mice *in vivo*.**

|  | 0 days | 14 days | 28 days |
|---|---|---|---|
| Control group (n=5) | 752-1 | 752-1 | 752-1 |
| Group 1 (n=5) | rTV-D8L-A56R | rTV-D8L-A56R-Δ-TK | rTV-D8L-A56R-Δ-TK |
| Group 2 (n=5) | rTV-D8L-A56R | rTV-D8L-A56R +rTV-TK | rTV-D8L-A56R +rTV-TK |

The results, as shown in Fig. 4, indicate that there was no significant difference between the two groups in terms of inducing binding antibodies. Group 1 showed a slightly better performance in inducing anti-p72 binding antibodies than Group 2, while Group 2 performed slightly better in inducing anti-p54 binding antibodies. There was no significant difference between the two groups in terms of T cell response induction.

From this example, it can be concluded that the provided recombinant vaccinia virus vector immunization combination against African swine fever virus, such as prime immunization with rTV-D8L-A56R followed by two booster doses of rTV-D8L-A56R + rTV-TK, can induce high titers of binding antibodies against p72, p54, and p30 in mice, as well as activate T cell responses against T antigen, p17, and penton. This combination shows promising application prospects in the prevention of African swine fever virus.It is noted that the more exogenous genes a vaccinia virus vector harbored, the more its amplification and replication capacity may be reduced. Considering the future industrial production costs, the more optimal recombinant vaccinia virus combination is the prime immunization with rTV-D8L-A56R followed by two booster doses of rTV-D8L-A56R + rTV-TK.

**Example 4. Further optimization of recombinant vaccinia virus**

Based on the aforementioned examples, we have identified an optimal recombinant vaccinia virus vector immunization combination, i.e., prime immunization with rTV-D8L-A56R followed by two booster doses of rTV-D8L-A56R + rTV-TK. Based on such scheme, we inserted a new immune exogenous gene, EP402R (SEQ ID NO: 97), which encodes the CD2v protein (i.e., EP402R peptide, SEQ ID NO: 98), into the K8R region of rTV-TK, resulting in the construction of rTV-TK-B646L-B602L-K8R-EP402R (hereinafter referred to as rTV-TK-K8R).

According to Experimental Method I, based on rTV-TK, the shuttle plasmid pK8R-EP402R (Fig. 5A), which carries the mCherry reporter gene, was recombined to generate a recombinant vaccinia virus, selected via mCherry screening. After multiple rounds of screening, rTV-TK-K8R was obtained (Fig. 5B).The genome was extracted as a template, and PCR verification was performed using primers K8R-F and K8R-R (SEQ ID NOs: 99 and 100). The results showed that the K8R region contained only a single band, with a size consistent with the expected 2690 bp (Fig. 5C). Western blot analysis confirmed the effective expression of the immunogen (Fig. 5D). This demonstrated the successful construction of the recombinant vaccinia virus rTV-TK-K8R, with EP402R correctly inserted into the K8R region of the vaccinia virus genome and properly expressed. Moreover, the results also confirmed that the incorporation of the immune exogenous gene EP402R into the vaccinia virus did not negatively affect its amplification and replication capacity. The resulted recombinant vaccinia virus can effectively load and express the contained immunogen. This combination takes full advantage of the large capacity of the vaccinia virus while ensuring that the exogenous gene loaded in a single vaccinia virus has a size appropriate for maintaining its amplification and replication ability.

**Example 5. Further optimized recombinant vaccinia virus combination induces immune response in mice *in vivo*.**

To explore the effects of adding a new immunogen on the immune response to the existing immunogens, we set up the following experiment, wherein all experimental groups of mice were primarily immunized with rTV-D8L-A56R. One group of mice was boosted with a combination of rTV-D8L-A56R and rTV-TK, while another group was boosted with a combination of rTV-D8L-A56R and rTV-TK-K8R. Antibody and T cell responses were evaluated 4 weeks after the final immunization (Fig. 6A), as described in the immunization in Table 3.

**Table 3. Immunogenicity of two African swine fever virus vaccinia virus vector vaccine combinations in BALB/c mice *in vivo*.**

|  | 0 days | 21 days | 42 days |
|---|---|---|---|
| Control group (n=6) | 752-1 | 752-1 | 752-1 |
| Group 1 (n=6) | rTV-D8L-A56R | rTV-D8L-A56R +rTV-TK | rTV-D8L-A56R +rTV-TK |
| Group 2 (n=6) | rTV-D8L-A56R | rTV-D8L-A56R +rTV-TK-K8R | rTV-D8L-A56R +rTV-TK-K8R |

The data on binding antibodies showed that the addition of the new immunogen did not impair the original immune response. There were no significant differences in the levels of p72, p54, and p30 binding antibodies between the two groups of mice. Overall, the combination of rTV-D8L-A56R and rTV-TK-K8R induced slightly higher p72 binding antibody levels (Fig. 6B). T cell response data also indicated that, although there were no significant differences between the two groups, the combination of rTV-D8L-A56R and rTV-TK-K8R induced a higher number of positive cells against each peptide library in the mice (Fig. 6C).

From the above example, it can be concluded that the optimized recombinant vaccinia virus vector immunization combination for African swine fever virus provided in the present disclosure, i.e., the scheme of prime immunization with rTV-D8L-A56R followed by two booster doses of rTV-D8L-A56R + rTV-TK-K8R, can effectively induce high titers of binding antibodies against p72, p54, and p30 in mice, as well as activate T cell responses against T antigen, p17, and penton. This combination has broad application prospects in the field of African swine fever virus prevention.

**Example 6. Evaluation of the protective effect of recombinant vaccinia virus combination against challenge in field pigs *in vivo*.**

The development of this vaccine aims at preventing and controlling African swine fever outbreaks. By screening African swine fever virus immunogen compositions, an African swine fever virus vaccine was constructed. Therefore, we further explored the immune response and protection from challenge induced by the recombinant vaccinia virus combination in pigs.

Ten 40-50 day old field pigs were randomly divided into two groups, with 4 pigs in the control group and 6 pigs in the experimental group. Immunization was performed according to Table 4. All groups received intramuscular injections, with a single immunization volume of 1 mL per pig. The first injection was administered at a dose of 2E8 PFU per pig, and the second injection at a dose of 5E8 PFU per pig. Four weeks after immunization, pigs were challenged with an intramuscular injection of 100 $HAD_{50}$ of the HLJ/18 strain, and observations continued for 21 days. The experimental procedure is shown in Fig.7A.

**Table 4. Immunogenicity of African swine fever virus vaccinia virus vector vaccine in field pigs *in vivo*.**

|  | 0 days | 28 days |
| --- | --- | --- |
| Control group (n=4) | 752-1 | 752-1 |
| Vaccine group (n= 6) | rTV-D8L-A56R +rTV-TK-K8R | rTV-D8L-A56R +rTV-TK-K8R |

All pigs were transferred to the P4 laboratory on Day 53 and challenged on Day 56, and observations were continued for 21 days after challenge. During the observation period, clinical scoring was performed daily for each pig, using the scoring criteria outlined in Table 5. Typical clinical symptoms of African swine fever virus infection were observed in the pigs, comprising decreased appetite, lethargy, and recumbency. Clinical symptoms appeared in all pigs starting on Day 4 post-challenge and progressively worsened. In the control group, clinical scores gradually increased and remained at their highest level from the day of challenge until the day of onset and death. In the vaccine group, clinical scores gradually decreased from approximately Day 10, indicating a gradual reduction in clinical symptoms, and the pigs essentially recovered to a healthy state by Day 21 post-challenge (Fig.7B). Body temperature gradually increased in both the control and vaccine groups after challenge. In the control group, body temperature remained above 40°C from Day 4 post-challenge, while in the vaccine group, body temperature gradually decreased from Day 7 post-challenge, dropping below 40°C after Day 14 and gradually returning to pre-challenge body temperature (Fig.7C, top panel). In the end, all four experimental pigs in the control group died within 15 days, while four out of the six experimental pigs in the vaccine group survived, with a survival rate of approximately 66.7% (Fig.7 C, bottom panel).

Generally, increasing the number of vaccinations can induce a stronger immune response. Compared with other examples, this example achieved a 66.7% protection rate with only two doses of the candidate vaccine before the challenge. Therefore, it is reasonable to envision that the protective effect of the vaccine combinations in other examples will be at least comparable to this example.

**Table 5. African Swine Fever Clinical Scoring System**

|  | Score 1 | Score 2 | Score 3 | Score 4 |
| --- | --- | --- | --- | --- |
| Feed intake status | Slight decrease in feed intake | Decrease in feed intake | Significantly decrease in feed intake | Refusal to eat |

(continued)

| | Score 1 | Score 2 | Score 3 | Score 4 |
|---|---|---|---|---|
| Mental status Recumbency tendency | Slight lethargy Lazy and unwilling to move | Mild lethargy responsive to stimulation | Significant lethargy sluggish to rise | Extreme lethargy unable to stand |
| Hemorrhage/c yanosis | According to the extent/area of hemorrhage and cyanosis | | | |
| Diarrhea | Mild diarrhea | pasty feces | watery feces | bloody feces |
| Joint swelling | Mild swelling | Swelling and lameness | - | Severe swelling and deformity/unable to stand |
| Other | Other clinical symptoms such as respiratory issues, rash, swelling, weight loss, vomiting, tear stains, and muscle tremors (Score 2 for each). | | | |

In summary, the recombinant vaccinia virus vector immunization combination against African swine fever virus provided in the present disclosure can effectively induce both humoral and cellular immune responses in experimental pigs and provide protection against a strong challenge with African swine fever virus.

All references cited herein are incorporated by reference in their entirety as though each reference was individually incorporated by reference. In addition, it should be understood that after reading the contents of the present application, those skilled in the art may make various changes or modifications to the present application, and these equivalent forms also fall within the scope defined by the appended claims of the present application.

**Appendix: Sequence information**

**Immunogenic sequences**

| SEQ ID NO for nucleotide sequence | SEQ ID NO for amino acid sequence | Sequence name |
|---|---|---|
| 1 | 2 | B646L (p72) |
| 3 | 4 | B602L (p72 chaperon) |
| 5 | 6 | H240R (penton) |
| 7 | 8 | B438L (p49) |
| 9 | 10 | C204L (p30) |
| 11 | 12 | D117L (p17) |
| 13 | 14 | E183L (p54) |
| 13+2n | 14+2n | T cell epitopes-n (n = 1~31) |
| 77 | 78 | T antigen (without DHFR) |
| 79 | 80 | T antigen (with DHFR) |
| 97 | 98 | EP402R (CD2v) |

**II. Other sequences**

| SEQ ID NO: | Sequence name | SEQ ID NO: | Sequence name |
|---|---|---|---|
| 81 | IRES | 90 | Peptide linker 2 |
| 82 | Loxp | 91 | Peptide linker 3 |
| 83 | PCR identification primer rTV-D8L-F | 92 | Peptide linker 4 |
| 84 | PCR identification primer rTV-D8L-R | 93 | Peptide linker 5 |

(continued)

| SEQ ID NO: | Sequence name | SEQ ID NO: | Sequence name |
|---|---|---|---|
| 85 | PCR identification primer rTV-A56R-F | 94 | Peptide linker 6 |
| 86 | PCR identification primer rTV-A56R-R | 95 | *cre* gene |
| 87 | PCR identification primer rTV-TK-F | 96 | cre polypeptide |
| 88 | PCR identification primer rTV-TK-R | 99 | PCR identification primer rTV-K8R-F |
| 89 | Peptide linker 1 | 100 | PCR identification primer rTV-K8R-R |

**III. Specific sequences**

SEQ ID NO: 1:*B646L* gene

atggcatcaggaggagctttttgtcttattgctaacgatgggaaggccgacaagattatattggcccaagacttgctgaatagcaggatctctaacattaaaaatgtga
acaaaagttatgggaaacccgatcccgaacccactttgagtcaaatcgaagaaacacatttggtgcattttaatgcgcattttaagccttatgttccagtagggtttgaa
tacaataaagtacgcccgcatacgggtacccccaccttgggaaacaagcttacctttggtattccccagtacggagacttttccatgatatggtgggccatcatatatt
gggtgcatgtcattcatcctggcaggatgctccgattcagggcacgtcccagatggggggccccatgggcagcttcaaacgtttcctcgcaacggatatgactgggac
aaccaaacaccccttagagggcgccgtttacacgcttgtagatccttttggaagaccccattgtacccggcacaaagaatgcgtaccgaaacttggtttactactgcgaa
tacccccggagaacgacttttatgaaaacgtaagattcgatgtaaatggaaattccctagacgaatatagttcggatgtcacaacgcttgtgcgcaaattttgcatcccag
gggataaaatgactggatataagcacttggttggccaggaggtatcggtggagggaaccagtggccctctcctatgcaacattcatgatttgcacaagccgcacca
aagcaaacctattcttaccgatgaaatgatacgcagcgaacgtgtagccataccaacccgaaatttctttcacagcattttcccgagaactctcacaatatccaaaca
gcaggtaaacaagatattactcctatcacggacgcaacgtatctggacataagacgtaatgttcattacagctgtaatggacctcaaaccccctaaatactatcagccc
cctcttgcgctctggattaagttgcgctttttggtttaatgagaacgtgaaccttgctattccctcagtatccattccttcggcgagcgctttatcaccataaagcttgcatc
gcaaaaggatttggtgaatgaattcctggactttttgtacgccagtcacgtttatagctggacgcccccagtagacgcaatatacgctttaaaccatggtttatcccag
gagtcattaatgaaatctcgctcacgaataatgaactttacatcaataacctgtttgtaacccctgaaatacacaacctttttgtaaaacgcgttcgctttcgctgatacgt
gtccataaaacgcaggtgacccacaccaacaataaccaccacgatgaaaaactaatgtctgctcttaaatggcccattgaatatatgtttataggattaaaacctacct
ggaacatctccgatcaaaatcctcatcaacaccgagattggcacaagttcggacatgttgttaacgccattatgcagcccactcaccacgcagagataagctttcag
gatagagatacagctcttccagacgcatgttcatctatatctgatattagccccgttacgtatccgatcacattacctattattaaaaacatttccgtaactgctcatggtat

caatcttatcgataaatttccatcaaagttctgcagctcttacataccccttccactacgaggcaatgcgattaaaaccccccgatgatccgggtgcgatgatgattacct
ttgctttgaagccacgggaggaataccaacccagtggtcatattaacgtatccagagcaagagaattttatattagttgggacacggattacgtggggtctatcactac
ggctgatcttgtggtatcggcatctgctattaactttcttcttcttcagaacggttcagctgtgctgcgttacagtacctaa

SEQ ID NO: 2:B646L polypeptide (p72)

MASGGAFCLIANDGKADKIILAQDLLNSRISNIKNVNKSYGKPDPEPTLSQIEETHLVHFNAHFKPYVPV
GFEYNKVRPHTGTPTLGNKLTFGIPQYGDFFHDMVGHHILGACHSSWQDAPIQGTSQMGAHGQLQTFP
RNGYDWDNQTPLEGAVYTLVDPFGRPIVPGTKNAYRNLVYYCEYPGERLYENVRFDVNGNSLDEYSSD
VTTLVRKFCIPGDKMTGYKHLVGQEVSVEGTSGPLLCNIHDLHKPHQSKPILTDENDTQRTCSHTNPKFL
SQHFPENSHNIQTAGKQDITPITDATYLDIRRNVHYSCNGPQTPKYYQPPLALWIKLRFWFNENVNLAIP
SVSIPFGERFITIKLASQKDLVNEFPGLFVRQSRFIAGRPSRRNIRFKPWFIPGVINEISLTNNELYINNLFVT
PEIHNLFVKRVRFSLIRVHKTQVTHTNNNHHDEKLMSALKWPIEYMFIGLKPTWNISDQNPHQHRDWH
KFGHVVNAIMQPTHHAEISFQDRDTALPDACSSISDISPVTYPITLPIIKNISVTAHGINLIDKFPSKFCSSYI
PFHYGGNAIKTPDDPGAMMITFALKPREEYQPSGHINVSRAREFYISWDTDYVGSITTADLVVSASAINF
LLLQNGSAVLRYST

SEQ ID NO: 3:*B602L* gene

atggcagaatttaatattgatgagcttctcaaaaacgtattggaggatccctctactgaaatatccgaagaaacgcttaaacagctttaccaaaggacgaacccttaca
aacagttcaaaaatgatagcagggtggcctttgctcttttacaaatttgcgggagcagtatattcgacgtcttataatgactagctttattggatatgtcttcaaagctctg
caggaatggatgccttcctattcaaaacctacccacacgaccaaaactcttctcagtgagctaataacgttagttgatactttgaaacaggaaactaatgatgttccctc
tgaatcggtagtaaatacaattttatctatagcagatagctgcaaaacccagacgcagaaaagcaaggaagctaaacaacgatcgatagcttttacgagaacattt
tgtgtttgatcctaatcttcatgctcaaagtgcgtatacttgtgcagataccaatgtagacacttgtgcaagcatgtgtgcagataccaatgtagacacctgtgcaagca
tgtgtgcagataccaatgtagatacctgtgcaagcacttgtacaagcacagaatacaccgatttagcagatcctgagcgcatccctttacacatcatgcaaaaaacatt
aaatgtgcctaatgagcttcaggccgatattgatgcaatcacccaaaccccacagggctataggggcagcagcccacatattacaaaatatagaacttcaccaaagc
attaaacatatgcttgaaaatccgagggcgtttaaacccattctctttaacacaaaaattactagatatctttcgcagcatattccacctcaggatactttttataagtggaa
ttattacattgaggataattacgaagagttgcgggccgctacggaaagcatctacccagaaaaacccgacctagagtttgccttcattatttatgatgtggtggatagc
agcaaccaacaaaaggttgatgaatttttattataaaatataaagaccagattttctcagaggtttcatccattcaattaggcaactggacactcctgggaagctttaaggc
caacagagagcgctacaattattttaatcaaaataatgaaataataaaacggattttggaccgtcatgaggaagacctaaagataggaaaagagatttacgaaatac
tatttaccacaaaaaagcaaaaaatatacaagaaactggccccggatgctccgggggctctccatctataattcaacctttcacacggatagcgggattaagggactgc
tttcctttaaggagctaaaaaacctagaaaaagcatctggaaatatcaaaaaagctcgagagtatgattttatagacgactgcgaagaaaaaattaagcaactgctta
gtaaagaaaatttaaccccgatgaagaaagcgagctgataaaaacaaaaaaacagttagataatgcgcttgaaatgctcaatgtgcctgatgatacgatacgggt
agatatgtgggtcaacaataataataaactcgaaaaagaaattttatatacaaaagcagaattgtaa

## SEQ ID NO: 4:B602L polypeptide (p72 chaperon)

MAEFNIDELLKNVLEDPSTEISEETLKQLYQRTNPYKQFKNDSRVAFCSFTNLREQYIRRLIMTSFIGYVF
KALQEWMPSYSKPTHTTKTLLSELITLVDTLKQETNDVPSESVVNTILSIADSCKTQTQKSKEAKTTIDSF
LREHFVFDPNLHAQSAYTCADTNVDTCASMCADTNVDTCASMCADTNVDTCASTCTSTEYTDLADPE
RIPLHIMQKTLNVPNELQADIDAITQTPQGYRAAAHILQNIELHQSIKHMLENPRAFKPILFNTKITRYLS
QHIPPQDTFYKWNYYIEDNYEELRAATESIYPEKPDLEFAFIIYDVVDSSNQQKVDEFYYKYKDQIFSEV
SSIQLGNWTLLGSFKANRERYNYFNQNNEIIKRILDRHEEDLKIGKEILRNTIYHKKAKNIQETGPDAPGL
SIYNSTFHTDSGIKGLLSFKELKNLEKASGNIKKAREYDFIDDCEEKIKQLLSKENLTPDEESELIKTKKQ
LDNALEMLNVPDDTIRVDMWVNNNNKLEKEILYTKAEL

## SEQ ID NO: 5:H240R gene

atggctgcaaacattattgcaacaagagccgtgccaaagatggccagcaaaaaagagcatcaatactgtctgctagactcccaggaaaagcgtcatgggcattatc
cctttcatttgaattaaagccttatgggcaaacaggcgcaaatatcataggagtacagggctcacttacccatgttatcaaaatgacagtatttccatttatgattcctttt
cctttacaaaaaactcatatagatgattttattggtggacgcatttatttattttttaaggaactggacatgcaagcagtttctgatgtaaatggaatgcaataccacttcga
gttcaaggttgttcctgtaagccccaaccaagtagagcttcttcctgtgaataataaatataaatttacatatgctataccggtagtgcaataccttaccccaatctttatg
atctttcgggaccgctagatttcccattagatactctttcggtccatgtggatatcctctccaatcatatacagcttcctatccaaaaccataacctaacaacgggtgatc
gtgtttttatttctggatataaacacctgcaaacgattgaattatgtaaaaataacaagattttttatcaaaaatataccgccgctttcatccgaaaaaataaaactatatatac
taaaaaatcgaatcagaattccgctatactttaaatctttaaaaacgtctaagtaa

## SEQ ID NO: 6:H240R polypeptide (penton)

MAANIIATRAVPKMASKKEHQYCLLDSQEKRHGHYPFSFELKPYGQTGANIIGVQGSLTHVIKMTVFPF
MIPFPLQKTHIDDFIGGRIYLFFKELDMQAVSDVNGMQYHFEFKVVPVSPNQVELLPVNNKYKFTYAIP
VVQYLTPIFYDLSGPLDFPLDTLSVHVDILSNHIQLPIQNHNLTTGDRVFISGYKHLQTIELCKNNKIFIKNI
PPLSSEKIKLYILKNRIRIPLYFKSLKTSK

## SEQ ID NO: 7:B438L gene

atgtatcatgattatgcttcaaagctgttagccgattataggtcagatccacctctttgggaaagcgatttgccgcgtcataaccggtattctgataatatactaaattccc ggtactgcggcaacaaaaatggagctgcgcctgtatacaatgaatacactaatagtcctgaaaaggctgaaaagggattgcaactcagtgatctacgtaactttctttt tatgctgaaccctcagcacaaaaacattggttacggccgatgcgcaagatctggagccgtacagctccattccaaaaaataaacttttttaatcattttaaaaatcatcgtc ctgcttttttcaaacccatacggaaaacctaattaggcgcaatgttgttcgtacggaaaaaaaaacatttccgcaggtggctagcttaaagggcacacagaaaaattgtc tgacccagccttcctcattgccttccttaaaaaaacccaaaaaatagttcggttccttctacgaggttcagcgagcacacaaaattttttcgtacgaggatcttcctaaac tcagaacaaagggtaccattaagcatgagcagcaccttggcgatcagatgccgggccagcactataacggatatattccgcacaaagacgtgtacaatattttgtgt ttagcgcacaatctacctgcgtcggtagaaaaagggattgcaggtagaggtattcctttaggaaatccacatgtcaaaccgaacatagagcaggaactcataaaatc caccagtacctacacggacgtgccgatgcttggtccccttccgcccaaagattcgcaacacggcagagaataccaggaattttccgcaaatagacatatgctacaa gtatctaatatactacattctgtgtttgctaatcattctataaagccacaaatcctagaagacataccggtactcaatgcacaactaacaagcataaagccggtaagccc attttaaacaaagcgtatcaaacccattatatggaaaatattgttaccttggtgccccggtttaagagtattgccaactactcaagccctataccgaattattctaaaaga aatagcggtcaggctgaatattttgatacctcaaagcaaacaatttcaagacacaataattatatacctaaatatacggggaggaattggagattccaaactagacagca cctttcctaaagatttcaacgcctcctcggtaccgttaacaagcgcgggaaaaggaccattccctgcggggagacaactcagcatgttgcatctcatctatatctccatc attgtga

SEQ ID NO: 8:B438L polypeptide (p49)

MYHDYASKLLADYRSDPPLWESDLPRHNRYSDNILNSRYCGNKNGAAPVYNEYTNSPEKAEKGLQLS
DLRNFSFMLNPQHKNIGYGDAQDLEPYSSIPKNKLFNHFKNHRPAFSTHTENLIRRNVVRTEKKTFPQV
ASLKGTQKNCLTQPSSLPSLKNPKNSSVPSTRFSEHTKFFSYEDLPKLRTKGTIKHEQHLGDQMPGQHY
NGYIPHKDVYNILCLAHNLPASVEKGIAGRGIPLGNPHVKPNIEQELIKSTSTYTDVPMLGPLPPKDSQH
GREYQEFSANRHMLQVSNILHSVFANHSIKPQILEDIPVLNAQLTSIKPVSPFLNKAYQTHYMENIVTLVP
RFKSIANYSSPIPNYSKRNSGQAEYFDTSKQTISRHNNYIPKYTGGIGDSKLDSTFPKDFNASSVPLTSAE
KDHSLRGDNSACCISSISPSL

SEQ ID NO: 9:*C204L* gene

atggattttattttaaatatatccatgaaaatggaggtcatcttcaaaacggatttaagatcatcttcacaagttgtgtttcatgcgggtagcctgtataattggttttctgttg agattatcaatagcggtagaattgttacgaccgctataaaaacattgcttagtactgttaagtatgatattgtgaaatctgctcgtatatatgcagggcaagggtatactg aacatcaggctcaagaagaatggaatatgattctgcatgtgctgtttgaagaggagacggaatcctcagcatcttcggagaacattcatgaaaaaaatgataatgaa accaatgaatgcacatcctcctttgaaacgttgtttgagcaagagccctcatcggaggtacctaaagactccaagctgtatatgcttcacaaaagactgtgcaacat attgaacaatatggaaaggcacctgatttaacaaggttattagagcacataattttattcaaaccatttatggaacccctctaaaggaagaagaaaaagaggtggtaa gactcatggttattaaacttttaaaaaaaaataa

SEQ ID NO: 10:C204L polypeptide (p30)

MDFILNISMKMEVIFKTDLRSSSQVVFHAGSLYNWFSVEIINSGRIVTTAIKTLLSTVKYDIVKSARIYAG
QGYTEHQAQEEWNMILHVLFEEETESSASSENIHEKNDNETNECTSSFETLFEQEPSSEVPKDSKLYMLA
QKTVQHIEQYGKAPDFNKVIRAHNFIQTIYGTPLKEEEKEVVRLMVIKLLKKK

SEQ ID NO: 11:*D117L* gene

atggacactgaaacgtctccactgctttctcataaacctgtcaacccgcgagggaattaaacaaagcacccaaggcctttagcccatacaatcgccaaatatcccgg aacaactgcgattctcctgggcattttgattttgctcattattattcttatcatcgttgccatcgtttactataaccggactattgactgcaagtcgagcatacctaaacctcc tcctagctactatgtacaacaacctgagcctcaccaccatttcccggtattctttagaaaaaggaaaaactccacctccctgcagtccacattccaagcgacgaaca attagctgaacttgcgcattcatga

SEQ ID NO: 12:D117L polypeptide (p17)

MDTETSPLLSHNLSTREGIKQSTQGLLAHTIAKYPGTTAILLGILILLIIILIIVAIVYYNRTIDCKSSIPKPPPS
YYVQQPEPHHHFPVFFRKRKNSTSLQSHIPSDEQLAELAHS

SEQ ID NO: 13:*E183L* gene

atggattctgaatttttttcaaccggtttatccgcggcattatggtgagtgtttgtcaccagtcactacaccaagcttcttctccacacatatgtatactattctcattgctatc
gtggtcttagtcatcattatcatcgttctaatctatctattctcttcaagaaagaaaaagctgctgctattgaggaggaagatatacagtttataaatccttatcaagatca
gcagtgggtagaagtcactccacaaccaggtacctctaaaccagctggagcgactacagcaagtgtaggcaagccagtcacgggcagaccggcaacaaacag
accagcaacaaacaaaccagttacggacaacccagttacggacagactagtcatggcaactggcggggccggcggccgcacctgcggccgcgagtgctcctgc
tcatccggctgagccttacacgacagtcactactcagaacactgcttcacaaacaatgtcggctattgaaaatttacgacaaagaaacacctatacgcataaagacct
agaaaactccttgtaa

SEQ ID NO: 14:E183L polypeptide (p54)

MDSEFFQPVYPRHYGECLSPVTTPSFFSTHMYTILIAIVVLVIIIIVLIYLFSSRKKKAAAIEEEDIQFINPYQ
DQQWVEVTPQPGTSKPAGATTASVGKPVTGRPATNRPATNKPVTDNPVTDRLVMATGGPAAAPAAASA

PAHPAEPYTTVTTQNTASQTMSAIENLRQRNTYTHKDLENSL

SEQ ID NO: 15:T-cell epitope-1 gene
tttatgcgcgaaattaaagtaaaggaagtgttatactttatacaatgcaggctgcccaagag

SEQ ID NO: 16:T-cell epitope-1 polypeptide
FMREIKVKEVLYFYTMQAAQE

SEQ ID NO: 17:T-cell epitope-2 gene

ggaagagttcccggttttttcaaaagaaatgctatttcaatatatacgctactttacagataatttgtgcttcatgatgcagtgcaaaagcatttataaggtgggaaacccctt
ttcctcagatgactaaa

ggaagagttcccggttttttcaaaagaaatgctatttcaatatatacgctactttacagataatttgtgcttcatgatgcagtgcaaaagcatttata
aggtgggaaacccctt ttcctcagatgactaaa

SEQ ID NO: 18:T-cell epitope-2 polypeptide
GRVPGFSKEMLFQYIRYFTDNLCFMMQCKSIYKVGNPFPQMTK

SEQ ID NO: 19:T-cell epitope-3 gene
acggaacaatttattagaaaccgcattaatttcattccgatttatgcgaaaaacgagacactggtatttgctctgcggagtttaaacaatagctgtgag

SEQ ID NO: 20:T-cell epitope-3 polypeptide
TEQFIRNRINFIPIYAKNETLVFALRSLNNSCE

SEQ ID NO: 21:T-cell epitope-4 gene
attgaacagtacccaacggataaaaaagaatatatggcgcttagcggttacaagcaaagctctatgacc

SEQ ID NO: 22:T-cell epitope-4 polypeptide
IEQYPTDKKEYMALSGYKQSSMT

SEQ ID NO: 23:T-cell epitope-5 gene
caaaataaccaaatagaaactattctggttcaaaaacgttacagccttgcttttttcagaatttattcattgtcattactctataaatgctaat

SEQ ID NO: 24:T-cell epitope-5 polypeptide
QNNQIETILVQKRYSLAFSEFIHCHYSINAN

SEQ ID NO: 25:T-cell epitope-6 gene
gaccgcatgtggtatcatatttggattgaaactccagtctacgaactataccacaaaaaataccaaaaatttaggaaa

SEQ ID NO: 26:T-cell epitope-6 polypeptide
DRMWYHIWIETPVYELYHKKYQKFRK

SEQ ID NO: 27:T-cell epitope-7 gene

tttaaaaaatctatgtcatactttgacggtaaaacagaatataagcatatctacttccttgcaatgttatgtaagtcgttggaggaaccc

SEQ ID NO: 28:T-cell epitope-7 polypeptide
FKKSMSYFDGKTEYKHIYFLAMLCKSLEEP

SEQ ID NO: 29:T-cell epitope-8 gene
gtggcccaaggtgaccatgtgcctgaccatatcaacatcctaaaaagaaaccaaatatttccgctgtttgcgcctaccccaagaggcgagggtagattt

SEQ ID NO: 30:T-cell epitope-8 polypeptide
VAQGDHVPDHINILKRNQIFPLFAPTPRGEGRF

SEQ ID NO: 31:T-cell epitope-9 gene
aatagcggaagaggatatatgcatgtaagattttctgctgttgtttcttacttaaatgcctttgatttaatagcggccgttaaaattattaaaaatgacagt

SEQ ID NO: 32:T-cell epitope-9 polypeptide
NSGRGYMHVRFSAVVSYLNAFDLIAAVKIIKNDS

SEQ ID NO: 33:T-cell epitope-10 gene
tcgcaaaataatgttgcagaagtggggcaaattgtgccggtacagcttgctaacagcagcgtgtactatatacctggcagacaacag

SEQ ID NO: 34:T-cell epitope-10 polypeptide
SQNNVAEVGQIVPVQLANSSVYYIPGRQQ

SEQ ID NO: 35:T-cell epitope-11 gene
tctaaaaaagacttcaagcagatatgcttttttgaaaaactttactatacctactctataagctcggat

SEQ ID NO: 36:T-cell epitope-11 polypeptide
SKKDFKQICFFEKLYYTYSISSD

SEQ ID NO: 37:T-cell epitope-12 gene
atgataattaacgtcatcatggatgtattttacgagacatattctttaccttataacatcaatctaacccgtgaacaaatt

SEQ ID NO: 38:T-cell epitope-12 polypeptide
MIINVIMDVFYETYSLPYNINLTREQI

SEQ ID NO: 39:T-cell epitope-13 gene
ggattaaataaaaagcgagaacttaaatactcctttcatatcatactttatacatactcagtattaaacaataatgaggcc

SEQ ID NO: 40:T-cell epitope-13 polypeptide
GLNKKRELKYSFHIILYTYSVLNNNEA

SEQ ID NO: 41:T-cell epitope-14 gene
gaccaagtgatgacaaagagcatagaggttcatgagtccattctctttgaagaactgcctgatactcaaaaa

SEQ ID NO: 42:T-cell epitope-14 polypeptide
DQVMTKSIEVHESILFEELPDTQK

SEQ ID NO: 43:T-cell epitope-15 gene
gaatcgatttttaatcaattcaatagcggcctgcacaaatactttgctccctcttttgccattttcatgtggatgctcgaaacggcaaattatgttata

SEQ ID NO: 44:T-cell epitope-15 polypeptide
ESIFNQFNSGLHKYFAPSFAIFMWMLETANYVI

SEQ ID NO: 45:T-cell epitope-16 gene
tgcttggacgcaaacctaggaaatcgaacgtataatattctacagcgttttcgcggggatgtccct

SEQ ID NO: 46:T-cell epitope-16 polypeptide
CLDANLGNRTYNILQRFRGDVP

SEQ ID NO: 47:T-cell epitope-17 gene
ttgaccaatgaacaattaatgacgaaactcgttgatgaatttgttcagtatgatttaagcgcgatcagctttgagtttaagcttaaaaagcccaataaaacggac

SEQ ID NO: 48:T-cell epitope-17 polypeptide
LTNEQLMTKLVDEFVQYDLSAISFEFKLKKPNKTD

SEQ ID NO: 49:T-cell epitope-18 gene
attgatattcatgaagtacgatatggagcctacacacttttcatgtatggttccctcgaaaacggttacaaagcagaagtaagg

SEQ ID NO: 50:T-cell epitope-18 polypeptide
IDIHEVRYGAYTLFMYGSLENGYKAEVR

SEQ ID NO: 51:T-cell epitope-19 gene

atggggtaccgcgagaaggaaaaagagtttgcaccatacattcgaatatttttaaaagcctgtatgagcgacgaaaagccattacttacttaaataatatgggctaca
acacggccgcggac

SEQ ID NO: 52:T-cell epitope-19 polypeptide
MGYREKEKEFAPYIRIFFKSLYERRKAITYLNNMGYNTAAD

SEQ ID NO: 53:T-cell epitope-20 gene
tcccgagaattaaaactacctcttacaagttggatacagcttcagcactattcctacgagcctcgcggcttggta

SEQ ID NO: 54:T-cell epitope-20 polypeptide
SRELKLPLTSWIQLQHYSYEPRGLV

SEQ ID NO: 55:T-cell epitope-21 gene

aatgttatccccgatcgcagagaggtaggaattctgtcatacacctcgctgtatgactgtatctactacgcgggaggacacaaggtatgcaatatgctcattgcctatg
ccatccatgatgaatacggccgtattgcttgc

SEQ ID NO: 56:T-cell epitope-21 polypeptide
NVIPDRREVGILSYTSLYDCIYYAGGHKVCNMLIAYAIHDEYGRIAC

SEQ ID NO: 57:T-cell epitope-22 gene
cgggatgaggcaaatagcctcatggccaagggtgaatctcttcactacgtctcctttcactttaacaatcgtctcgtg

SEQ ID NO: 58:T-cell epitope-22 polypeptide
RDEANSLMAKGESLHYVSFHFNNRLV

SEQ ID NO: 59:T-cell epitope-23 gene
cacgaaaaagtgcttctgtctatgaaagccatgtctacactatgcgccgaggtgaatgaatacctgcga

SEQ ID NO: 60:T-cell epitope-23 polypeptide
HEKVLLSMKAMSTLCAEVNEYLR

SEQ ID NO: 61:T-cell epitope-24 gene
atggtgttaatagagttttaacaggtttcttctatttatatggaaagagactgttttccatt

SEQ ID NO: 62:T-cell epitope-24 polypeptide
MVLIEFLTGFFYLYGKRLFSI

SEQ ID NO: 63:T-cell epitope-25 gene
tgtcggaagaacattttcttccttccaaatgattttagcaagcataccctacaatggctgggattatattggaaagagcatggctccgtc

SEQ ID NO: 64:T-cell epitope-25 polypeptide
CRKNIFFLPNDFSKHTLQWLGLYWKEHGSV

SEQ ID NO: 65:T-cell epitope-26 gene
ctggccgtgaaaaatcaatcttccaggaaaactttgaacttgttgctatcttacataaattacaaggtgaaaaatgttaaaaagctg

SEQ ID NO: 66:T-cell epitope-26 polypeptide
LAVKNQSSRKTLNLLLSYINYKVKNVKKL

SEQ ID NO: 67:T-cell epitope-27 gene

acagaaagaattacttatctcaaacagatagtgcacaccataaaatatgaaagtggaaggcgatttttggtagacatcattcacagcatttaccaaagttactcactaaa
acacgaagatattctt

SEQ ID NO: 68:T-cell epitope-27 polypeptide
TERITYLKQIVHTIKYESGRRFLVDIIHSIYQSYSLKHEDIL

SEQ ID NO: 69:T-cell epitope-28 gene
gcgcaggttaaatatacccttcaagagcttatttcctactgcagcgctctaaccattttacattatgactattcaaccctt

SEQ ID NO: 70:T-cell epitope-28 polypeptide
AQVKYTLQELISYCSALTILHYDYSTL

SEQ ID NO: 71:T-cell epitope-29 gene
gcacgaaaccccttttgtatcccaaagtatgtccttgaactattacttttatgaacctgaactaagc

SEQ ID NO: 72:T-cell epitope-29 polypeptide
ARNPFVSQSMSLNYYFYEPELS

SEQ ID NO: 73:T-cell epitope-30 gene
gtgtatgaaaagggtaagaccatgcccgtggaattttactatgaacaagagtttgat

SEQ ID NO: 74:T-cell epitope-30 polypeptide
VYEKGKTMPVEFYYEQEFD

SEQ ID NO: 75:T-cell epitope-31 gene
attaccaaggaagaaatcatgcaagcctatgctttggagtatgccatgtat

SEQ ID NO: 76:T-cell epitope-31 polypeptide
ITKEEIMQAYALEYAMY

SEQ ID NO: 77:T antigen gene(without DHFR)

atgtttatgcgcgaaattaaagtaaaggaagtgttatactttttatacaatgcaggctgcccaagagggaagagttcccggtttttcaaaagaaatgctatttcaatatata cgctactttacagataaatttgtgcttcatgatgcagtgcaaaagcatttataaggtgggaaacccttttcctcagatgactaaaacggaacaatttattagaaaccgcatt aatttcattccgatttatgcgaaaaacgagacactggtatttgctctgcggagtttaaacaatagctgtgagattgaacagtacccaacggataaaaaagaatatatgg cgcttagcggttacaagcaaagctctatgacccaaaataaccaaatagaaactattctggttcaaaaacgttacagccttgcttttcagaatttattcattgtcattactct ataaatgctaatgaccgcatgtggtatcatatttggattgaaactccagtctacgaactataccacaaaaaataccaaaaatttaggaaatttaaaaaatctatgtcatac tttgacggtaaaacagaatataagcatatctacttccttgcaatgttatgtaagtcgttggaggaacccgtggcccaaggtgaccatgtgcctgaccatatcaacatcc taaaaagaaaccaaatatttccgctgtttgcgcctaccccaagaggcgagggtagatttaatagcggaagaggatatatgcatgtaagattttctgctgttgtttcttact taaatgcctttgatttaatagcggccgttaaaattattaaaaatgacagttcgcaaaataatgttgcagaagtggggcaaattgtgccggtacagcttgctaacagcag cgtgtactatatacctggcagacaacagtctaaaaaagacttcaagcagatatgcttttttgaaaaactttactatacctactctataagctcggatatgataattaacgtc atcatggatgtatttacgagacatattctttaccttataacatcaatctaacccgtgaacaaattggattaaataaaaagcgagaacttaaatactcctttcatatcatactt tatacatactcagtattaaacaataatgaggccgaccaagtgatgacaaagagcatagaggttcatgagtccattctctttgaagaactgcctgatactcaaaaagaat cgattttaatcaattcaatagcggcctgcacaaatactttgctccctcttttgccatttcatgtggatgctcgaaacggcaaattatgttatatgcttggacgcaaaccta ggaaatcgaacgtataatattctacagcgtttcgcgggggatgtcccttttgaccaatgaacaattaatgacgaaactcgttgatgaatttgttcagtatgatttaagcgcg atcagctttgagtttaagcttaaaaagcccaataaaacggacattgatattcatgaagtacgatatggagcctacacactttttcatgtatggttccctcgaaaacggttac aaagcagaagtaaggatggggtaccgcgagaaggaaaaagagtttgcaccatacattcgaatattttttaaaagcctgtatgagcgacgaaaagccattacttactt aaataatatgggctacaacacgggccgcggactcccgagaattaaaactacctcttacaagttggatacagcttcagcactattcctacgagcctcgcggcttggtaa atgttatccccgatcgcagagaggtaggaattctgtcatacacctcgctgtatgactgtatctactacgcggggaggacacaaggtatgcaatatgctcattgcctatgc catccatgatgaatacggccgtattgcttgccgggatgaggcaaatagcctcatggccaaggggtgaatctcttcactacgtctcctttcactttaacaatcgtctcgtgc acgaaaaagtgcttctgtctatgaaagccatgtctacactatgcgccgaggtgaatgaataacctgcgaatggtgttaatagagtttttaacaggtttcttctatttatatgg aaagagactgttttccatttgtcggaagaacatttcttccttccaaatgattttagcaagcatacccctacaatggctgggattatattggaaagagcatggctccgtcct ggccgtgaaaaatcaatcttccaggaaaactttgaacttgttgctatcttacataaattacaaggtgaaaaatgttaaaaagctgacagaaagaattacttatctcaaac agatagtgcacaccataaaatatgaaagtggaaggcgattttttggtagacatcattcacagcatttaccaaagttactcactaaaacacgaagatattcttgcgcaggt taaatataccccttcaagagcttatttcctactgcagcgctctaaccattttacattatgactattcaacccttgcacgaaacccctttgtatcccaaagtatgtccttgaacta ttacttttatgaacctgaactaagcgtgtatgaaaagggtaagaccatgcccgtgaatttttactatgaacaagagtttgatattaccaaggaagaaatcatgcaagcc tatgctttggagtatgccatgtattga

SEQ ID NO: 78:T antigen polypeptide (without DHFR)

MFMREIKVKEVLYFYTMQAAQEGRVPGFSKEMLFQYIRYFTDNLCFMMQCKSIYKVGNPFPQMTKTE
QFIRNRINFIPIYAKNETLVFALRSLNNSCEIEQYPTDKKEYMALSGYKQSSMTQNNQIETILVQKRYSLA
FSEFIHCHYSINANDRMWYHIWIETPVYELYHKKYQKFRKFKKSMSYFDGKTEYKHIYFLAMLCKSLE
EPVAQGDHVPDHINILKRNQIFPLFAPTPRGEGRFNSGRGYMHVRFSAVVSYLNAFDLIAAVKIIKNDSSQ
NNVAEVGQIVPVQLANSSVYYIPGRQQSKKDFKQICFFEKLYYTYSISSDMIINVIMDVFYETYSLPYNIN
LTREQIGLNKKRELKYSFHIILYTYSVLNNNEADQVMTKSIEVHESILFEELPDTQKESIFNQFNSGLHKY
FAPSFAIFMWMLETANYVICLDANLGNRTYNILQRFRGDVPLTNEQLMTKLVDEFVQYDLSAISFEFKL
KKPNKTDIDIHEVRYGAYTLFMYGSLENGYKAEVRMGYREKEKEFAPYIRIFFKSLYERRKAITYLNNM
GYNTAADSRELKLPLTSWIQLQHYSYEPRGLVNVIPDRREVGILSYTSLYDCIYYAGGHKVCNMLIAYAI

HDEYGRIACRDEANSLMAKGESLHYVSFHFNNRLVHEKVLLSMKAMSTLCAEVNEYLRMVLIEFLTGF
FYLYGKRLFSICRKNIFFLPNDFSKHTLQWLGLYWKEHGSVLAVKNQSSRKTLNLLLSYINYKVKNVKK
LTERITYLKQIVHTIKYESGRRFLVDIIHSIYQSYSLKHEDILAQVKYTLQELISYCSALTILHYDYSTLARN
PFVSQSMSLNYYFYEPELSVYEKGKTMPVEFYYEQEFDITKEEIMQAYALEYAMY

SEQ ID NO: 79:T antigen gene(with DHFR)

atgtttatgcgcgaaattaaagtaaaggaagtgttatacttttatacaatgcaggctgcccaagagggaagagttcccggttttcaaaagaaatgctatttcaatatata
cgctactttacagataatttgtgcttcatgatgcagtgcaaaagcatttataaggtgggaaacccttttcctcagatgactaaaacggaacaatttattagaaaccgcatt
aatttcattccgatttatgcgaaaaacgagacactggtatttgctctgcggagtttaaacaatagctgtgagattgaacagtacccaacggataaaaaagaatatatgg
cgcttagcggttacaagcaaagctctatgacccaaaataaccaaatagaaactattctggttcaaaaacgttacagccttgcttttcagaatttattcattgtcattactct
ataaatgctaatgaccgcatgtggtatcatatttggattgaaactccagtctacgaactataccacaaaaaataccaaaaatttaggaaatttaaaaaatctatgtcatac
tttgacggtaaaacagaatataagcatatctacttccttgcaatgttatgtaagtcgttggaggaacccgtggcccaaggtgaccatgtgcctgaccatatcaacatcc
taaaaagaaaccaaatatttccgctgtttgcgcctaccccaagaggcgagggtagatttaatagcggaagaggagatatatgcatgtaagattttctgctgttgtttcttact
taaatgcctttgatttaatagcggccgttaaaattattaaaaatgacagttcgcaaaataatgttgcagaagtggggcaaattgtgccggtacagcttgctaacagcag
cgtgtactatatacctggcagacaacagtctaaaaaagacttcaagcagatatgcttttttgaaaaactttactatacctactctataagctcggatatgataattaacgtc
atcatggatgtattttacgagacatattctttaccttataacatcaatctaacccgtgaacaaattggattaaataaaaagcgagaacttaaatactcctttcatatcatactt
tatacatactcagtattaaacaataatgaggccgaccaagtgatgacaaagagcatagaggttcatgagtccattctctttgaagaactgcctgatactcaaaaagaat
cgattttaatcaattcaatagcggcctgcacaaatactttgctccctcttttgccattttcatgtggatgctcgaaacggcaaattatgttatatgcttggacgcaaaccta
ggaaatcgaacgtataatattctacagcgtttcgcggggatgtcccctttgaccaatgaacaattaatgacgaaactcgttgatgaatttgttcagtatgatttaagcgcg
atcagctttgagtttaagcttaaaaagcccaataaaacggacattgatattcatgaagtacgatatggagcctacacacttttcatgtatggttccctcgaaaacggttac
aaagcagaagtaaggatggggtaccgcgagaaggaaaaagagtttgcaccatacattcgaatatttttaaaagcctgtatgagcgacgaaaagccattacttactt
aaataatatgggctacaacacgggccgcggactcccgagaattaaaactacctcttacaagttggatacagcttcagcactattcctacgagcctcgcggcttggtaa
atgttatccccgatcgcagagaggtaggaattctgtcatacacctcgctgtatgactgtatctactacgcgggaggacacaaggtatgcaatatgctcattgcctatgc
catccatgatgaatacggccgtattgcttgccgggatgaggcaaatagcctcatggccaaggggtgaatctcttcactacgtctcctttcactttaacaatcgtctcgtgc
acgaaaaagtgcttctgtctatgaaagccatgtctacactatgcgccgaggtgaatgaataacctgcgaatggtgttaatagagtttttaacaggtttcttctatttatatgg
aaagagactgttttccatttgtcggaagaacatttcttccttccaaatgattttagcaagcatacccctacaatggctgggattatattggaaagagcatggCtccgtcct
ggccgtgaaaaatcaatcttccaggaaaactttgaacttgttgctatcttacataaaattacaaggtgaaaaatgttaaaaagctgacagaaagaattacttatctcaaac
agatagtgcacaccataaaatatgaaagtggaaggcgattttttggtagacatcattcacagcatttaccaaagttactcactaaaacacgaagatattcttgcgcaggt
taaatatacccttcaagagcttatttcctactgcagcgctctaaccattttacattatgactattcaacccttgcacgaaacccctttgtatcccaaagtatgtccttgaacta
ttacttttatgaacctgaactaagcgtgtatgaaaagggtaagaccatgcccgtggaattttactatgaacaagagtttgatattaccaaggaagaaatcatgcaagcc
tatgctttggagtatgccatgtatggcggcggggggaagcatgatcagcctgatcgccgccctggccgtggactacgtgctgggcatggagaacgccatgccctgg
aacctgcccgccgacctggcctggttcaagcggaacaccctgaacaagcccgtgatcatgggcagacacacctgggagtccatcggcagacccctgcccggc
agaaagaacatcatcctatcgtcccagccctccaccgacgaccgggtgacctgggtgaagagcgtggacgaggccatcgccgctgcggcgacgtgcccgag
atcatggtgatcggcggcggcagagtgatcgagcagttcctgcccaaggcccagaagctgtacctgacccacctggacgccgaggtggagggcgacacccac
ttccccgactacgagcccgacgactgggagtccgtgttcagcgagttccacgacgccgacgcccagaacagccactcctactgcttcgagatcctggagagaag
gtag

<u>SEQ ID NO: 80:T antigen polypeptide (with DHFR)</u>

MFMREIKVKEVLYFYTMQAAQEGRVPGFSKEMLFQYIRYFTDNLCFMMQCKSIYKVGNPFPQMTKTE
QFIRNRINFIPIYAKNETLVFALRSLNNSCEIEQYPTDKKEYMALSGYKQSSMTQNNQIETILVQKRYSLA
FSEFIHCHYSINANDRMWYHIWIETPVYELYHKKYQKFRKFKKSMSYFDGKTEYKHIYFLAMLCKSLE
EPVAQGDHVPDHINILKRNQIFPLFAPTPRGEGRFNSGRGYMHVRFSAVVSYLNAFDLIAAVKIIKNDSSQ
NNVAEVGQIVPVQLANSSVYYIPGRQQSKKDFKQICFFEKLYYTYSISSDMIINVIMDVFYETYSLPYNIN
LTREQIGLNKKRELKYSFHIILYTYSVLNNNEADQVMTKSIEVHESILFEELPDTQKESIFNQFNSGLHKY
FAPSFAIFMWMLETANYVICLDANLGNRTYNILQRFRGDVPLTNEQLMTKLVDEFVQYDLSAISFEFKL
KKPNKTDIDIHEVRYGAYTLFMYGSLENGYKAEVRMGYREKEKEFAPYIRIFFKSLYERRKAITYLNNM
GYNTAADSRELKLPLTSWIQLQHYSYEPRGLVNVIPDRREVGILSYTSLYDCIYYAGGHKVCNMLIAYAI
HDEYGRIACRDEANSLMAKGESLHYVSFHFNNRLVHEKVLLSMKAMSTLCAEVNEYLRMVLIEFLTGF
FYLYGKRLFSICRKNIFFLPNDFSKHTLQWLGLYWKEHGSVLAVKNQSSRKTLNLLLSYINYKVKNVKK
LTERITYLKQIVHTIKYESGRRFLVDIIHSIYQSYSLKHEDILAQVKYTLQELISYCSALTILHYDYSTLARN
PFVSQSMSLNYYFYEPELSVYEKGKTMPVEFYYEQEFDITKEEIMQAYALEYAMYGGGGSMISLIAALA
VDYVLGMENAMPWNLPADLAWFKRNTLNKPVIMGRHTWESIGRPLPGRKNIILSSQPSTDDRVTWVKS
VDEAIAACGDVPEIMVIGGGRVIEQFLPKAQKLYLTHLDAEVEGDTHFPDYEPDDWESVFSEFHDADAQ
NSHSYCFEILERR

<u>SEQ ID NO: 81:IRES</u>

tccctcccccccccctaacgttactggccgaagccgcttggaataaggccggtgtgcgtttgtctatatgttattttccaccatattgccgtcttttggcaatgtgagggc
ccggaaacctggccctgtcttcttgacgagcattcctaggggtctttcccctctcgccaaaggaatgcaaggtctgttgaatgtcgtgaaggaagcagttcctctgga

agcttcttgaagacaaacaacgtctgtagcgacccttgcaggcagcggaaccccccacctggcgacaggtgcctctgcggccaaaagccacgtgtataagatac
acctgcaaaggcggcacaaccccagtgccacgttgtgagttggatagttgtggaaagagtcaaatggctctcctcaagcgtattcaacaaggggctgaaggatgc
ccagaaggtaccccattgtatgggatctgatctgggggcctcggtgcacatgctttacatgtgtttagtcgaggttaaaaaaacgtctaggccccccgaaccacgggg
acgtggttttcctttgaaaaacacgatgataa

SEQ ID NO: 82:Loxp
ATAACTTCGTATAGCATACATTATACGAAGTTAT

SEQ ID NO: 83:PCR identification primer rTV-D8L-F
GACGACGACAATAGTGTTCTG

SEQ ID NO: 84:PCR identification primer rTV-D8L-R
CATTAGATCCGCCAATACG

SEQ ID NO: 85:PCR identification primer rTV-A56R-F
TCGAGGTTATTGGTTGTGGATC

SEQ ID NO: 86:PCR identification primer rTV-A56R-R
TTAGTTTCCTCTGGAAGCAC

SEQ ID NO: 87:PCR identification primer rTV-TK-F
CAAGGTCCCTATTGTTACAG

SEQ ID NO: 88:PCR identification primer rTV-TK-R
CGTATATGTGTACCGGGAGC

SEQ ID NO: 89:Peptide linker 1
GGGGS

SEQ ID NO: 90:Peptide linker 2
GSAGSAAGSGEFGSG

SEQ ID NO: 91:Peptide linker 3
EAAAK

SEQ ID NO: 92:Peptide linker 4
EFPKPSTPPGSSGGAP

SEQ ID NO: 93:Peptide linker 5
KESGSVSSEQLAQFRSLD

SEQ ID NO: 94:Peptide linker 6
EGKSSGSGSESKST

SEQ ID NO: 95: *cre* gene

atgtctaatctcctgacagtccaccagaatctgcccgctctcccgtggatgcaacttccgatgaggtgcggaagaatctcatggatatgtttcgcgatagacaggct
tttagcgagcacacttggaagatgctcctgtccgtgtgtaggtcttgggccgcttggtgtaagctcaataaccgcaagtggttcccagccgagcccgaggatgtgcg
cgactacctgctgtacctccaggctaggggactggcagtcaagactattcagcagcacctcggccagctgaacatgctccaccgccggtccggcctccctagacc
atctgattccaatgccgtgtcactcgtcatgagacgcattagaaaggagaacgtcgacgctggcgagcgcgctaagcaggccctcgcctttgagcggaccgacttt
gatcaggtgagatccctcatggagaactctgatcggtgccaggacattcggaatctcgcctttctgggaattgcatacaacaccctcctgcgcattgcagagatcgc
taggatcagagtcaaggatatttcacgcactgacggagggcgcatgctcatccacatcggacgcacaaagacactcgtgtctactgctggcgtcgagaaggctct
gtcactgggagtgacaaagctggtcgagcggtggattagcgtgtccggcgtcgccgacgaccctaataattacctgtttgtagagtgcggaagaacggcgtcgct
gccccttctgcaacttcacagctgtctactagggcactggagggaatttcgaggcaacccacagactcatttacgggggccaaggacgatagcgggcagcggtac
ctggcttggtccggccactcagctagagtgggagccgcccgcgatatggctagagccggggtctctatcccagagattatgcaggcagggggatggaccaatgt
gaatattgtgatgaactacatcagaaacctcgattctgagaccggggctatggtgcgcctgctcgaggacggcgattga

SEQ ID NO: 96:cre polypeptide

MSNLLTVHQNLPALPVDATSDEVRKNLMDMFRDRQAFSEHTWKMLLSVCRSWAAWCKLNNRKWFPA
EPEDVRDYLLYLQARGLAVKTIQQHLGQLNMLHRRSGLPRPSDSNAVSLVMRRIRKENVDAGERAKQA
LAFERTDFDQVRSLMENSDRCQDIRNLAFLGIAYNTLLRIAEIARIRVKDISRTDGGRMLIHIGRTKTLVST
AGVEKALSLGVTKLVERWISVSGVADDPNNYLFCRVRKNGVAAPSATSQLSTRALEGIFEATHRLIYGA
KDDSGQRYLAWSGHSARVGAARDMARAGVSIPEIMQAGGWTNVNIVMNYIRNLDSETGAMVRLLED
GD

SEQ ID NO: 97:*EP402R* gene

atgatcattctcatctttctgatcttctctaacatcgtgctctccatcgactactgggtgagcttcaacaagaccatcatcttggacagcaacatcacaaacgacaacaac
gacatcaacggcgtgtcttggaacttcttcaacaacagcttcaacacactggccacttgtggaaaggccggaaacttctgcgagtgctctaactacagcacctctatc
tacaacatcaccaacaactgcagcctgaccatcttccctcacaacgacgtgttcgacaccacctaccaggtggtgtggaaccagatcatcaactacaccatcaagct
gctgacaccagctacaccaccaaacatcacctacaactgcaccaacttcttgatcacatgcaagaagaacaacggaaccaacaccaacatctacctgaacatcaa
cgacaccttcgtgaagtacaccaacgagtccatcttggagtacaactggaacaactcaaacatcaacaacttcaccgccacatgcatcatcaacaacaccatcagc
accagcaacgagaccactctgatcaactgcacctacctcacactctccagcaactacttctacaccttcttcaagctgtactacatacctctcagcatcatcatcggcat
cacaatcagcattctgctgatctctatcatcaccttcttgagcctgagaaagcggaagaagcacgtggaagagatcgagagtccgccacctgagagcaacgaaga
agagcagtgtcagcatgatgacaccaccagcatccacgagccttccaagagagcctctgcttccaaagccttacagccggtaccagtacaacacaccaatctac
tacatgagaccaagcacacagccactgaacccctttcccactgcctaagccttgtccgcctccgaaaccttgtccaccacctaagccatgtccacctccaaagccatg
cccttctgcagagagctactctccaccgaaacctctgccaagcattccactgctgccaaacatccctccactgagcacacagaacatcagcctgattcacgtggatc
gcatcattggaggcggtggaagctga

SEQ ID NO: 98:EP402R polypeptide (CD2v)

MIILIFLIFSNIVLSIDYWVSFNKTIILDSNITNDNNDINGVSWNFFNNSFNTLATCGKAGNFCECSNYSTSI
YNITNNCSLTIFPHNDVFDTTYQVVWNQIINYTIKLLTPATPPNITYNCTNFLITCKKNNGTNTNIYLNIND
TFVKYTNESILEYNWNNSNINNFTATCIINNTISTSNETTLINCTYLTLSSNYFYTFFKLYYIPLSIIIGITISIL
LISIITFLSLRKRKKHVEEIESPPPESNEEEQCQHDDTTSIHEPSPREPLLPKPYSRYQYNTPIYYMRPSTQP
LNPFPLPKPCPPPKPCPPPKPCPPPKPCPSAESYSPPKPLPSIPLLPNIPPLSTQNISLIHVDRIIGGGGS

SEQ ID NO: 99:PCR identification primer rTV-K8R-F
GAACATACAGTTTGCACTCATG

SEQ ID NO: 100:PCR identification primer rTV-K8R-R
CGCATACTATATGCGATGATTG

**Claims**

1. An immunogenic composition comprising

    (A) An antibody immunogen group containing immunogens derived from structural proteins of African swine fever virus; and
    (B) A recombinant T-cell immunogen group containing immunogens derived from non-structural proteins of African swine fever virus.

2. The immunogenic composition according to claim 1, wherein the African swine fever virus is selected from circulating strains of African swine fever virus, and/or from multiple strains of African swine fever virus with different genotypes and serotypes,
for example, the African swine fever virus is one or more African swine fever virus strains selected from the group consisting of Pig/HLJ/18, China/Guangxi/2019/domestic pig, CN201801, China/Jilin/2018/boar, Pig/Hubei/628/2020, Pig/Liaoning/LC/2020, Pig/Hebei/Q3/2020, and Georgia 2007/1.

3. The immunogenic composition according to claim 1, wherein the antibody immunogen group comprises one or more full-length or extracellular fragments of structural proteins derived from African swine fever virus,

for example, the antibody immunogen group comprises one or more proteins or fragments selected from the group consisting of B646L, B602L, H240R, B438L, C204L, D117L, E183L, and EP402R; or any combination thereof, for example,

a combination of D117L and E183L (D117L-E183L),

a combination of H240R, B438L, and C204L (H240R-B438L-C204L),

a combination of B646L and B602L (B646L-B602L),

a combination of D117L, E183L, H240R, B438L, and C204L (D117L-E183L-H240R-B438L-C204L),

a combination of D117L, E183L, H240R, B438L, C204L, B646L, and B602L (D117L-E183L-H240R-B438L-C204L-B646L-B602L),

a combination of B646L, B602L, and EP402R (B646L-B602L-EP402R);

wherein the proteins or fragments in any of the combinations are directly linked or linked via one or more linkers, for example, a linker selected from SEQ ID NOs: 89-94, IRES, P2A, T2A, E2A, and F2A.

4.  The immunogenic composition according to claim 3, wherein the B646L, B602L, H240R, B438L, C204L, D117L, E183L, and EP402R are peptide segments having the amino acid sequences set forth in SEQ ID NOs: 2, 4, 6, 8, 10, 12, 14, and 98, respectively, or peptide segments having at least 80% sequence identity or homology with the amino acid sequences set forth in SEQ ID NOs: 2, 4, 6, 8, 10, 12, 14, and 98; and/or

the B646L, B602L, H240R, B438L, C204L, D117L, E183L, and EP402R are encoded by nucleic acid molecules having the nucleotide sequences as set forth in SEQ ID NOs: 1, 3, 5, 7, 9, 11, 13, and 97, respectively, or by nucleic acid molecules having at least 80 % sequence identity or homology with the nucleotide sequences as set forth in SEQ ID NOs: 1, 3, 5, 7, 9, 11, 13, and 97; and/or

optionally, one or more linkers independently selected from the group consisting of IRES, P2A, T2A, E2A, and F2A are further comprised between the B646L, B602L, H240R, B438L, C204L, D117L, E183L, and EP402R.

5.  The immunogenic composition according to claim 1, wherein the recombinant T- cell immunogen group comprises one or more non- structural proteins or fragments thereof derived from African swine fever virus, for example, the recombinant T-cell immunogen group comprises

(a) one or more T- cell epitopes comprised in non-structural proteins selected from the group consisting of F334L, H359L, D250R, D339L, F1055L, G1211R, A528, and MGF -505-7R proteins; or any combination of the T-cell epitopes comprised in the aforesaid non-structural proteins; or fragments having at least 80% sequence identity with the aforementioned combinations of the T-cell epitopes; and

(b) optionally, one or more linkers located between the peptide elements, such as one or more linkers selected from SEQ ID NOs: 89-94, IRES, P2A, T2A, E2A, and F2A.

6.  The immunogenic combination according to claim 5, wherein the recombinant T- cell immunogen group comprises at least a combination of T- cell epitopes comprised in F334L, H359L, D250R, D339L, F1055L, G1211R, A528, and MGF-505-7R proteins; and/or

the recombinant T- cell immunogen group comprises

(i) at least 8, 10, 20, 30, or all peptide segments selected from the group consisting of peptide segments having the amino acid sequence set forth in SEQ ID NOs: 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, or peptide segments having at least 80% sequence identity with any of the aforementioned peptide segments; and/or

(ii) a peptide segment having the amino acid sequence set forth in SEQ ID NO: 78 or 80, or a peptide segment having at least 80 % sequence identity with any of the aforementioned peptides; and/or

the recombinant T-cell immunogen group comprises

(i') at least 8, 10, 20, 30, or all nucleic acid molecules selected from the group consisting of nucleic acid molecules having nucleotide sequences set forth in SEQ ID NOs: 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, or 75, or nucleic acid molecules having at least 80% sequence identity with any one of the aforementioned nucleic acid molecules; and/or

(ii') a nucleic acid molecule having the nucleotide sequence set forth in SEQ ID NO: 77 or 79, or a nucleic acid molecule having at least 80% sequence identity with any one of the aforementioned nucleic acid molecules.

7. The immunogenic composition according to claim 3, wherein the recombinant T-cell immunogen group is linked or mixed with the antibody immunogen group, for example, the recombinant T-cell immunogen group is mixed or linked with B646L, B602L, H240R, B438L, C204L, D117L, E183L, EP402R or any combination thereof (e.g., a combination of D117L and E183L), for example, each is independently linked via one or more linkers selected from SEQ ID NOs: 89-94, IRES, P2A, T2A, E2A, and F2A; and/or

   wherein the recombinant T cell immunogen group is linked or mixed with the antibody immunogen group to form one or more combinations selected from the group consisting of D117L-E183L-T antigen, D117L-E183L-T antigen-H240R-B438L-C204L, D117L-E183L-T antigen-H240R-B438L-C204L-B646L-B602L, or any combination thereof; and/or a combination of the aforementioned three immunogens comprising T antigens or combination thereof and one or more immunogens selected from the group consisting of D117L-E183L, H240R-B438L-C204L, B646L-B602L, D117L-E183L-H240R-B438L-C204L, D117L-E183L-H240R-B438L-C204L-B646L-B602L, B646L-B602L-EP402R;
   for example, a combination of D117L-E183L-T antigen-H240R-B438L-C204L and D117L-E183L-T antigen-H240R-B438L-C204L-B646L-B602L; a combination of D117L-E183L-T antigen-H240R-B438L-C204L and B646L-B602L; a combination of D117L-E183L-T antigen-H240R-B438L-C204L and B646L-B602L-EP402R.

8. A recombinant T- cell immunogenic peptide comprising

   (a) one or more T- cell epitopes comprised in non-structural proteins derived from African swine fever virus (ASFV) selected from the group consisting of F334L, H359L, D250R, D339L, F1055L, G1211R, A528, and MGF -505-7R proteins; or any combination of the T-cell epitopes comprised in the aforesaid non-structural proteins; or fragments having at least 80% sequence identity with the aforementioned combinations of the T-cell epitopes; and
   (b) optionally, one or more linkers located between the peptide elements, such as one or more linkers selected from SEQ ID NOs: 89-94, IRES, P2A, T2A, E2A, and F2A.

9. The recombinant T- cell immunogenic peptide according to claim 8, wherein

   the recombinant T- cell immunogenic peptide comprises at least a combination of T-cell epitopes comprised in F334L, H359L, D250R, D339L, F1055L, G1211R, A528, and MGF-505-7R proteins; and/or
   the recombinant T-cell immunogenic peptide comprises

   (i) at least 8, 10, 20, 30, or all peptide segments selected from the group consisting of peptide segments having the amino acid sequence set forth in SEQ ID NOs: 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, or peptide segments having at least 80% sequence identity with any of the aforementioned peptide segments; and/or
   (ii) a peptide segment having the amino acid sequence set forth in SEQ ID NO: 78 or 80, or a peptide segment having at least 80 % sequence identity with any of the aforementioned peptides; and/or

   the recombinant T-cell immunogenic peptide comprises

   (i') at least 8, 10, 20, 30, or all nucleic acid molecules selected from the group consisting of nucleic acid molecules having nucleotide sequences set forth in SEQ ID NOs: 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, and 75, or nucleic acid molecules having at least 80% sequence identity with any one of the aforementioned nucleic acid molecules; and/or
   (ii') a nucleic acid molecule having the nucleotide sequence set forth in SEQ ID NO: 77 or 79, or a nucleic acid molecule having at least 80% sequence identity with any one of the aforementioned nucleic acid molecules.

10. A nucleic acid molecule encoding the immunogenic composition according to any one of claims 1-7, or the recombinant T-cell immunogenic peptide according to any one of claims 8-9.

11. A vector or host cell comprising the nucleic acid molecule according to claim 10,

   for example, the vector is selected from mRNA vectors, DNA plasmid vectors (e.g., shuttle plasmid vectors), recombinant viral vectors, and recombinant bacterial vectors; wherein the mRNA vector is selected from linear, circular, and self-replicating vectors; and the recombinant viral vector is selected from vaccinia virus (e.g., Tiantan strain, North American vaccine strain, Wyeth derivative strain, Listeria strain, Ankara derivative strain, Copenhagen strain, and New York strain), adenovirus (e.g., adenovirus types 5, 11, 26, 35, 63, and 68), adeno-associated virus, herpes simplex virus, measles virus, enterovirus, reovirus, rhabdovirus, flavivirus, influenza virus, parainfluenza virus, respiratory syncytial virus, and poliovirus vectors;

for example, the host cells are mammalian or insect cells, such as HEK293, HeLa, K562, CHO, NS0, SP2/0, PER.C6, Vero, RD, BHK, HT 1080, A549, Cos-7, ARPE-19, and MRC-5 cells; High Five, Sf9, Se301, SeIZD2109, SeUCR1, Sf9, Sf900+, Sf21, BTI-TN-5B1-4, MG-1, Tn368, HzAm1, BM-N, Ha2302, Hz2E5, and Ao38.

12. An African swine fever vaccine, comprising

(I) one or more immunogenic components or precursors thereof selected from the group consisting of one or more of the immunogenic compositions according to any one of claims 1-7, the recombinant T-cell immunogenic peptides according to any one of claims 8-9, the nucleic acid molecules according to claim 10, and the vectors and host cells according to claim 11; and
(II) one or more pharmaceutically or veterinarily acceptable carriers or excipients or delivery systems.

13. The African swine fever vaccine according to claim 12, wherein

the African swine fever vaccine is an mRNA vaccine, a DNA vaccine, a viral vector vaccine (e.g., a vaccinia virus vaccine), or a recombinant protein vaccine; and/or
the carrier or excipient or delivery system is selected from lipid delivery systems, lipid-like delivery systems, polymer delivery systems or combinations thereof, such as loaded onto lipid nanoparticles (e.g., a combination of cationic lipids, structural lipids, auxiliary lipids and stabilizing lipids), polyurethane (PAA), poly-$\beta$-amino ester (PBAE), polyethyleneimine (PEI), and lipid-encapsulated polymer micelles; and/or
the vaccine also comprises an adjuvant or is used in combination with an adjuvant, for example, the adjuvant is selected from aluminum adjuvant, cholera toxin and subunits thereof, oligodeoxynucleotides, manganese ion adjuvant, colloidal manganese adjuvant, Freund's adjuvant, MF59 adjuvant, QS-21 adjuvant, Poly I:C and other TLR ligands, GM-CSF, IL-2, IL-3, IL-7, IL-11, IL-12, IL-18, and IL-21; and/or
the vaccine is suitable for one or more administration or delivery methods selected from the group consisting of respiratory nebulization, nasal drops, oral administration, direct injection (e.g., intravenous, subcutaneous, intradermal, intramuscular), and mucosal administration; and/or
the vaccine is in a form suitable for administration of two or more drugs or vaccines in combination, such as combined or sequential vaccination; and/or
the vaccine is a monovalent or multivalent vaccine, and its form is suitable for sequential or combined immunization.

14. The African swine fever vaccine according to claim 12 or 13, wherein the vaccine is a recombinant vaccinia virus (rTV) vaccine, for example,

(a) the rTV vaccine comprises the following elements: a vaccinia virus vector; a vaccinia virus recombination site; a vaccinia virus shuttle plasmid; a vaccinia virus promoter; and a selection marker; and/or
(b) the recombinant vaccinia virus is selected from one or more recombinant vaccinia virus that have one or more of the following immunogenic fragments inserted: rTV- D117L -E183L-T antigen, rTV- H240R-B438L -C204L, rTV-B646L-B602L, rTV-D117L-E183L-T antigen-H240R-B438L-C204L, rTV -D117L-E183L-T antigen-H240R-B438L-C204L- B646L-B602L, rTV-B646L-B602L, or rTV-B646L-B602L-EP402R, wherein the T antigen represents the recombinant T-cell immunogen group according to any one of claims 1-7; and/or
(c) the recombination site of the vaccinia virus is derived from a combination of one or more non-replication-essential gene sites of the vaccinia strain used, such as J2R, A56R, K8R, C6L, K1L, B13R, A46R, N1L, and K8R; and/or
(d) the vaccinia virus promoter is derived from a strong promoter that initiates viral protein expression in vaccinia virus, such as pE/L, p7.5, pH5, and different truncated forms of each strong promoter; the selection markers comprise reporter genes such as LacZ, eGFP, mCherry, BFP, and ZsGreen.

15. A product for the prevention and/or treatment of African swine fever, comprising one or more of the immunogenic compositions according to any one of claims 1-7, the recombinant T- cell immunogenic peptides according to any one of claims 8-9, the nucleic acid molecules according to claim 10, the vectors or host cells according to claim 11, and the vaccines according to any one of claims 12-14.

16. The product according to claim 15, wherein the product is a vaccine kit comprising one or more doses of the same or different vaccines according to any one of claims 12-14,

for example, the product comprises one or more doses of a combination of recombinant vaccinia virus vaccines selected from the group consisting of rTV-D117L-E183L-T antigen, rTV- H240R-B438L-C204L, rTV-B646L-B602L, rTV-D117L-E183L-T antigen-H240R-B438L-C204L, rTV-D117L-E183L-T antigen-H240R-B438L-C204L-B646L-B602L, rTV-B646L-B602L, and rTV-B646L-B602L-EP402R;

preferably, any combination of rTV- D117L-E183L-T antigen-H240R-B438L-C204L, rTV -D117L-E183L-T antigen-H240R-B438L-C204L-B646L-B602L, rTV-B646L-B602L, and rTV-B646L-B602L-EP402R;

more preferably, a combination of one dose of rTV- D117L-E183L-T antigen-H240R-B438L-C204L with one or two doses of rTV- D117L-E183L-T antigen-H240R-B438L-C204L-B646L-B602L, or rTV-rTV-B646L-B602L, or rTV-B646L-B602L-EP402R, or a combination of two or three doses of rTV- D117L-E183L-T antigen-H240R-B438L-C204L-B646L-B602L and rTV-B646L-B602L or rTV-B646L-B602L-EP402R.

Fig. 1

Fig. 2

**Fig. 3**

**Fig. 4**

Fig. 5

**Fig. 6**

**Fig. 7**

## INTERNATIONAL SEARCH REPORT

| International application No. |
|---|
| **PCT/CN2024/117421** |

### A. CLASSIFICATION OF SUBJECT MATTER

A61K39/12(2006.01)i; C07K14/01(2006.01)i; C12N15/34(2006.01)i; A61P31/20(2006.01)i

According to International Patent Classification (IPC) or to both national classification and IPC

### B. FIELDS SEARCHED

Minimum documentation searched (classification system followed by classification symbols)

IPC:A61K; C07K; C12N; A61P

Documentation searched other than minimum documentation to the extent that such documents are included in the fields searched

Electronic data base consulted during the international search (name of data base and, where practicable, search terms used)

CJFD, CNKI, CNABS, CNTXT, ENTXT, ENTXTC, VEN, BING, WEB OF SCIENCE, PUBMED, NCBI, EMBL, STN, 中国专利生物序列检索数据库, China Patents Biological Sequence Search Database: 非洲猪瘟病毒, African swine fever virus, ASFV, ASF, 非洲猪瘟, 结构蛋白, structural protein, 非结构蛋白, non-structural, non structural, T cell antigen, T 细胞表位, T cell epitope, T细胞抗原, T抗原, T细胞免疫原, T淋巴细胞表位, T lymphocyte epitope, CTL表位, T-cell, T细胞, 表位, epitope, IFN-γ, 疫苗, vaccine, F334L, H359L, D250R, D339L, F1055L, G1211R, A528, A528R, MGF_505-7R, MGF505-7R, MGF-505-7R, B646L, p72, pB602L, B602L, H240R, pH240R, B438L, p49, C204L, p30, E183L, p54, EP402R, CD2v, pEP402R, CP204L, P32, P17, 接头, linker, 2A肽, ires, 自切割肽, 连接肽, 连接臂, SEQ ID NOs: 1-96

### C. DOCUMENTS CONSIDERED TO BE RELEVANT

| Category* | Citation of document, with indication, where appropriate, of the relevant passages | Relevant to claim No. |
|---|---|---|
| X | CN 110904127 A (STICHTING WAGENINGEN RES) 24 March 2020 (2020-03-24) description, paragraphs 10 and 50-100, and table 1 | 1-3, 5, 7-8, 10-16 |
| Y | CN 110904127 A (STICHTING WAGENINGEN RES) 24 March 2020 (2020-03-24) description, paragraphs 10 and 50-100, and table 1 | 4 |
| X | JANCOVICH, J. K. et al. "Immunization of Pigs by DNA Prime and Recombinant Vaccinia Virus Boost To Identify and Rank African Swine Fever Virus Immunogenic and Protective Proteins" *Journal of Virology Journal Homepage.* Vol. 92, No. 8, 31 January 2018 (2018-01-31), document number e02219-17, pages 1-14 abstract, pages 11, paragraphs 2-3 and paragraph 5 to page 12, paragraph 5, table 1, and figures 1-3 | 1-3, 5, 7-8, 10-16 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 December 2024** | **11 December 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

# EP 4 785 951 A1

## INTERNATIONAL SEARCH REPORT

| International application No. |
|---|
| **PCT/CN2024/117421** |

**C.    DOCUMENTS CONSIDERED TO BE RELEVANT**

| Category* | Citation of document, with indication, where appropriate, of the relevant passages | Relevant to claim No. |
|---|---|---|
| Y | CN 113365656 A (KANSAS STATE UNIVERSITY RESEARCH FOUNDATION) 07 September 2021 (2021-09-07)<br>abstract, claims 1-15, and description, paragraphs 24-26 | 4 |
| Y | CN 113543801 A (BOEHRINGER INGELHEIM VETMEDICA GMBH) 22 October 2021 (2021-10-22)<br>abstract, claims 1-22, and description, paragraph 94, and embodiment 2 | 4 |
| X | CN 113831394 A (ACADEMY OF MILITARY MEDICAL SCIENCES OF THE PLA ACADEMY OF MILITARY SCIENCE) 24 December 2021 (2021-12-24)<br>claims 4 and 6-10 | 1-3, 5, 7, 10-12, 15 |
| X | CN 114606250 A (JILIN AGRICULTURAL UNIVERSITY) 10 June 2022 (2022-06-10)<br>claims 1-11, and description, paragraphs 5 and 226-239 | 1-3, 5, 7, 10-12, 15 |
| PX | CN 118121693 A (ZHEJIANG DINGCHI BIOLOGICAL PRODUCTS CO., LTD. et al.) 04 June 2024 (2024-06-04)<br>description, paragraph 25, and table 2 (group 2) | 1-3, 5, 7-8, 10-16 |
| A | US 2023192777 A1 (EPIVAX, INC.) 22 June 2023 (2023-06-22)<br>entire document | 1-16 |

Form PCT/ISA/210 (second sheet) (July 2022)

43

## INTERNATIONAL SEARCH REPORT

International application No.

**PCT/CN2024/117421**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2024/117421**

| Patent document cited in search report | | | Publication date (day/month/year) | Patent family member(s) | | | Publication date (day/month/year) |
|---|---|---|---|---|---|---|---|
| CN | 110904127 | A | 24 March 2020 | AU | 2019345214 | A1 | 08 April 2021 |
| | | | | CL | 2021000636 | A1 | 01 October 2021 |
| | | | | ZA | 202101615 | B | 26 April 2023 |
| | | | | WO | 2020060403 | A2 | 26 March 2020 |
| | | | | WO | 2020060403 | A3 | 14 May 2020 |
| | | | | TW | 202033538 | A | 16 September 2020 |
| | | | | TWI | 777093 | B | 11 September 2022 |
| | | | | EP | 3852798 | A2 | 28 July 2021 |
| | | | | CO | 2021004188 | A2 | 20 May 2021 |
| | | | | US | 2022047694 | A1 | 17 February 2022 |
| | | | | KR | 20210065128 | A | 03 June 2021 |
| | | | | JP | 2022500050 | A | 04 January 2022 |
| | | | | JP | 7548902 | B2 | 10 September 2024 |
| | | | | BR | 112021005079 | A2 | 08 June 2021 |
| | | | | PH | 12021550580 | A1 | 29 November 2021 |
| | | | | CA | 3112800 | A1 | 26 March 2020 |
| | | | | MX | 2021003175 | A | 14 May 2021 |
| CN | 113365656 | A | 07 September 2021 | KR | 20210092762 | A | 26 July 2021 |
| | | | | WO | 2020102370 | A1 | 22 May 2020 |
| | | | | US | 2022031831 | A1 | 03 February 2022 |
| CN | 113543801 | A | 22 October 2021 | TW | 202102258 | A | 16 January 2021 |
| | | | | WO | 2020193688 | A2 | 01 October 2020 |
| | | | | WO | 2020193688 | A3 | 08 April 2021 |
| | | | | KR | 20210145221 | A | 01 December 2021 |
| | | | | CA | 3130664 | A1 | 01 October 2020 |
| | | | | MX | 2021011687 | A | 22 October 2021 |
| | | | | EP | 3946445 | A2 | 09 February 2022 |
| | | | | JP | 2022525920 | A | 20 May 2022 |
| | | | | JP | 7572372 | B2 | 23 October 2024 |
| | | | | AU | 2020247131 | A1 | 11 November 2021 |
| | | | | US | 2023277648 | A1 | 07 September 2023 |
| | | | | US | 11998596 | B2 | 04 June 2024 |
| | | | | EA | 202192570 | A1 | 20 January 2022 |
| | | | | US | 2020306360 | A1 | 01 October 2020 |
| | | | | US | 11628214 | B2 | 18 April 2023 |
| CN | 113831394 | A | 24 December 2021 | None | | | |
| CN | 114606250 | A | 10 June 2022 | None | | | |
| CN | 118121693 | A | 04 June 2024 | None | | | |
| US | 2023192777 | A1 | 22 June 2023 | MX | 2022007217 | A | 12 September 2022 |
| | | | | WO | 2021119424 | A1 | 17 June 2021 |
| | | | | CA | 3161222 | A1 | 17 June 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HEALTH W O F A**. AFRICAN SWINE FEVER (ASF). *Situation Report*, 2024, vol. 55 (R)
- **OURA C A** ; **DENYER M** ; **TAKAMATSU H et al.** In vivo depletion of CD8+ T lymphocytes abrogates protective immunity to African swine fever virus[J. *Journal of General Virology*, 2005, vol. 86 (9), 2445-2450
- **WU K** ; **LIU J** ; **WANG L et al.** Current state of global African swine fever vaccine development under the prevalence and transmission of ASF in China[J].. *Vaccines*, 2020, vol. 8 (3), 531
- **CHEN W** ; **ZHAO D** ; **HE X et al.** A seven-gene-deleted African swine fever virus is safe and effective as a live attenuated vaccine in pigs[J].. *Sci China Life Sci*, 2020, vol. 63 (5), 623-634
- **GOMEZ-PUERTAS P** ; **RODRIGUEZ F** ; **OVIEDO J M et al.** Neutralizing antibodies to different proteins of African swine fever virus inhibit both virus attachment and internalization[J. *Journal of virology*, 1996, vol. 70 (8), 5689-5694
- **LACASTA A** ; **BALLESTER M** ; **MONTEAGUDO P L et al.** Expression library immunization can confer protection against lethal challenge with African swine fever virus[J].. *Journal of virology*, 2014, vol. 88 (22), 13322-13332
- **GOATLEY L C** ; **REIS A L** ; **PORTUGAL R et al.** A pool of eight virally vectored African swine fever antigens protect pigs against fatal disease[J].. *Vaccines*, 2020, vol. 8 (2), 234
- Molecular Cloning: A Laboratory Manual. Cold Spring Harbor Laboratory Press, 1989